# EUROPÄISCHE PATENTSCHRIFT

(11) **EP 1 694 681 B1**
(45) Veröffentlichungstag und Bekanntmachung des Hinweises auf die Patenterteilung: **12.03.2008**
(21) Anmeldenummer: 04803610.7
(22) Anmeldetag: 08.12.2004
(51) Int. Cl.: C07D 487/04, A01N 43/90

(54) **PYRAZOLOPYRIMIDINE**
PYRAZOLOPYRIMIDINES
PYRAZOLOPYRIMIDINES

(30) Priorität: 10.12.2003 DE 10357568
(43) Veröffentlichungstag der Anmeldung: 30.08.2006
(73) Patentinhaber: Bayer CropScience AG, 40789 Monheim (DE)
(72) Erfinder: GEBAUER, Olaf, 51377 Leverkusen (DE); HEINEMANN, Ulrich, 42799 Leichlingen (DE); HERRMANN, Stefan, 40764 Langenfeld (DE); GAYER, Herbert, 40789 Monheim am Rhein (DE); HILLEBRAND, Stefan, 41462 Neuss (DE); ELBE, Hans-Ludwig, 42329 Wuppertal (DE); EBBERT, Ronald, 90475 Nünberg (DE); WACHENDORFF-NEUMANN, Ulrike, 56566 Neuwied (DE); DAHMEN, Peter, 41470 Neuss (DE); KUCK, Karl-Heinz, 40764 Langenfeld (DE)
(86) Internationale Anmeldenummer: PCT/EP2004/013931
(87) Internationale Veröffentlichungsnummer: WO 2005/056556

(56) Entgegenhaltungen:
- FR-A- 2 794 745
- US-A- 5 965 561
- US-A1- 2002 049 318
- NOVINSON T ET AL: "Synthesis and antifungal properties of certain 7-alkylaminopyrazolo(1,5-a)pyrimidines" JOURNAL OF MEDICINAL CHEMISTRY, AMERICAN CHEMICAL SOCIETY, US, Bd. 20, Nr. 2, 1977, Seiten 296-299, XP002970049 ISSN: 0022-2623

## Beschreibung

Die Erfindung betrifft Pyrazolopyrimidine, mehrere Verfahren zu deren Herstellung und deren Verwendung zur Bekämpfung von unerwünschten Mikroorganismen.

Es ist bereits bekannt geworden, dass bestimmte Pyrazolopyrimidine fungizide Eigenschaften besitzen (vergleiche DE-A 3 130 633 oder FR-A 2 794 745).

Da sich aber die ökologischen und ökonomischen Anforderungen an moderne Fungizide laufend erhöhen, beispielsweise was Wirkspektrum, Toxizität, Selektivität, Aufwandmenge, Rückstandsbildung und günstige Herstellbarkeit angeht, und außerdem z.B. Probleme mit Resistenzen auftreten können, besteht die ständige Aufgabe, neue Fungizide zu entwickeln, die zumindest in Teilbereichen Vorteile gegenüber den bekannten aufweisen.

Es wurden nun neue Pyrazolopyrimidine der Formel 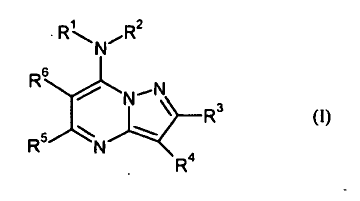 in welcher
R¹ für Wasserstoff, gegebenenfalls substituiertes Alkyl, gegebenenfalls substituiertes Alkenyl,
gefunden.

Weiterhin wurde gefunden, dass sich Pyrazolopyrimidine der Formel (I) herstellen lassen, indem man

### a) Cyano-Verbindungen der Formel

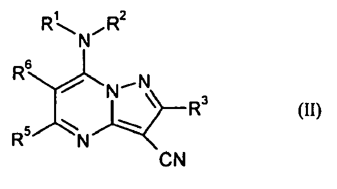

in welcher
R¹, R², R³, R⁵ und R⁶ die oben angegebenen Bedeutungen haben,
entweder
- α): mit Säuren und Wasser gegebenenfalls in Gegenwart eines Verdünnungsmittels umsetzt,
- β): mit Hydroxylamin oder einem Hydroxylammonium-Salz in Gegenwart eines Verdünnungsmittels und gegebenenfalls in Gegenwart eines Katalysators umsetzt,
oder
- γ): mit Ammoniumchlorid in Gegenwart einer Base und in Gegenwart eines Verdünnungsmittels umsetzt,
oder

### b) Carbonyl-Verbindungen der Formel

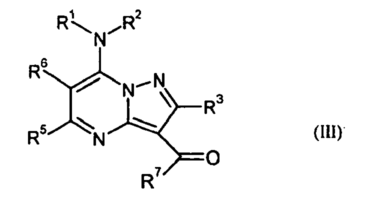

in welcher
R¹, R², R³, R⁵, R⁶ und R⁷ die oben angegebenen Bedeutungen haben,
mit Amino-Verbindungen der Formel

H₂N-R⁸ (IV)

in welcher
- R⁸: die oben angegebenen Bedeutungen hat,
in Gegenwart eines Verdünnungsmittels und gegebenenfalls in Gegenwart eines Katalysators umsetzt, wobei die Amino-Verbindungen der Formel (IV) auch in Form ihrer Säureadditions-Salze eingesetzt werden können.

Schließlich wurde gefunden, dass sich die Pyrazolopyrimidine der Formel (I) sehr gut zur Bekämpfung von unerwünschten Mikroorganismen eignen. Sie zeigen vor allem eine starke fungizide Wirksamkeit und lassen sich sowohl im Pflanzenschutz als auch im Materialschutz verwenden.

Die erfindungsgemäßen Verbindungen können je nach Substitutionsmuster gegebenenfalls als Mischungen verschiedener möglicher isomerer Formen, insbesondere von Stereoisomeren, wie E-und Z-, threo- und erythro-, sowie optischen Isomeren, gegebenenfalls aber auch in Form von Tautomeren vorliegen. Ist R⁶ an beiden Atomen, die der Bindungsstelle benachbart sind, ungleich substituiert, können die betreffenden Verbindungen in einer besonderen Form der Stereoisomerie vorliegen und zwar als Atropisomere.

Die erfindungsgemäßen Pyrazolopyrimidine sind durch die Formel (I) allgemein definiert. Bevorzugt sind diejenigen Stoffe der Formel (I), in denen
- R¹: für Wasserstoff oder einen Rest der Formel 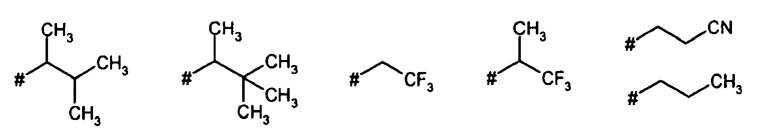 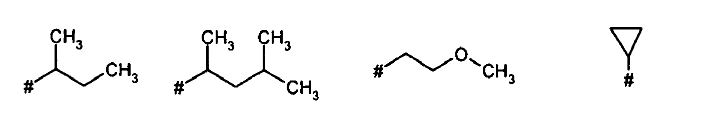 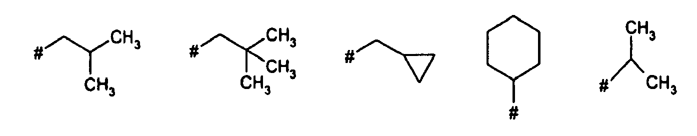 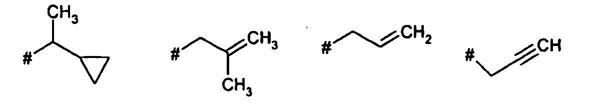 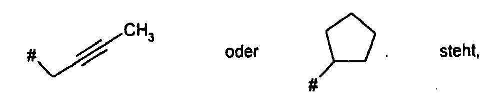 wobei # die Anknüpfungsstelle markiert,
- R²: für Wasserstoff, Methyl, Ethyl oder Propyl steht, oder
- R¹ und R²: gemeinsam mit dem Stickstoffatom, an das sie gebunden sind, für Pyrrolidinyl, Piperidinyl, Morpholinyl, Thiomorpholinyl, Piperazinyl, 3,6-Dihydro-1(2H)-piperidinyl oder Tetrahydro-1(2H)-pyridazinyl stehen, wobei diese Reste durch 1 bis 3 Fluoratome, 1 bis 3 Methylgruppen und/oder Trifluormethyl substituiert sein können,
oder
- R¹ und R²: gemeinsam mit dem Stickstoffatom, an das sie gebunden sind, für einen Rest der Formel 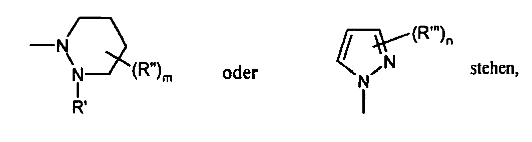 worin
R' für Wasserstoff oder Methyl steht,
R" für Methyl, Ethyl, Fluor, Chlor oder Trifluormethyl steht,
m für die Zahlen 0, 1, 2 oder 3 steht, wobei R" für gleiche oder verschiedene Reste steht, wenn m für 2 oder 3 steht,
R'" für Methyl, Ethyl, Fluor, Chlor oder Trifluormethyl steht und
n für die Zahlen 0, I, 2 oder 3 steht, wobei R'" für gleiche oder verschiedene Reste steht, wenn n für 2 oder 3 steht,
- R³: für Wasserstoff, Fluor, Chlor, Brom, Iod, Methyl, Ethyl, Isopropyl, Cyclopropyl, Cyclobutyl, Cyclopentyl, Cyclohexyl, Trifluormethyl, I-Trifluormethyl-2,2,2-trifluorethyl oder Heptafluorisopropyl steht,
- R⁴: für einen Rest der Formel 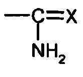 steht, worin
X für ein Sauerstoffatom, eine HN-Grupe, eine HO-N-Gruppe oder Z-O-N= steht, wobei Z für Alkyl oder Arylalkyl steht, oder
- R⁴: für einen Rest der Formel 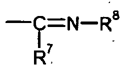 steht, worin
R⁷ für Wasserstoff, Methyl oder Ethyl steht und
- R⁸: für Alkyl mit 1 oder 2 Kohlenstoffatomen steht, wobei jeder dieser Alkyl-Reste substituiert sein kann durch Methoxy, Ethoxy, Methylcarbonyl, Ethylcarbonyl, Methoxycarbonyl oder Ethoxycarbonyl, oder
- R⁸: für Phenyl steht, das einfach bis dreifach, gleichartig oder verschieden substituiert sein kann durch Methyl, Ethyl, Methoxy, Ethoxy, Fluor, Chlor, Brom, Nitro und/oder Trifluormethyl, oder
- R⁸: für Phenylamino steht, das einfach bis dreifach, gleichartig oder verschieden substitueirt sein kann durch Methyl, Ethyl, Methoxy, Ethoxy, Fluor, Chlor, Brom, Nitro und/oder Trifluormethyl,
- R⁵: für Fluor, Chlor, Brom, Methoxy, Ethoxy, Methylthio, Methylsulfinyl oer Methylsulfonyl steht, und
- R⁶: für Phenyl steht, das einfach bis dreifach, gleichartig oder verschieden substitiert sein kann durch Fluor, Chlor, Brom, Cyano, Nitro, Formyl, Methyl, Ethyl, n- oder i-Propyl, n-, i-, s- oder t-Butyl, Allyl, Propargyl, Methoxy, Ethoxy, n- oder i-Propoxy, Methylthio, Ethylthio, n- oder i-Propylthio, Methylsulfinyl, Ethylsulfinyl, Methylsulfonyl, Ethylsulfonyl, Allyloxy, Propargyloxy, Trifluormethyl, Trifluorethyl, Difluormethoxy, Trifluormethoxy, Difluorchlormethoxy, Trifluorethoxy, Difluormethylthio, Difluorchlormethylthio, Trifluormethylthio, Trifluormethylsulfinyl, Trifluormethylsulfonyl, Trichlorethinyloxy, Trifluorethinyloxy, Chlorallyloxy, Iodpropargyloxy, Methylamino, Ethylamino, n- oder i-Propylamino, Dimethylamino, Diethylamino, Acetyl, Propionyl, Acetyloxy, Methoxycarbonyl, Ethoxycarbonyl, Hydroximinomethyl, Hydroximinoethyl, Methoximinomethyl, Ethoximinomethyl, Methoximinoethyl, Ethoximinoethyl, Cyclopropyl, Cyclobutyl, Cyclopentyl oder Cyclohexyl,
in 2,3-Position verknüpftes 1,3-Propandiyl, Methylendioxy (-O-CH₂-O-) oder 1,2-Ethylendioxy (-O-CH₂-CH₂-O-), wobei diese Reste einfach oder mehrfach, gleichartig oder verschieden substituiert sein können durch Fluor, Chlor, Methyl, Ethyl, n-Propyl, i-Propyl und/oder Trifluormethyl.

Eine besonders bevorzugte Gruppe erfindungsgemäßer Verbindungen sind Pyrazolopyrimidine der Formel (I), in denen
- R¹, R², R³ und R⁴: die zuvor angegebenen bevorzugten Bedeutungen haben,
- R⁵: für Fluor, Chlor, Brom, Methoxy oder Methylthio steht und
- R⁶: für 2,4-, 2,5- oder 2,6-disubstituiertes Phenyl, oder 2-substituiertes Phenyl oder für 2,4,6-trisubstituiertes Phenyl steht, wobei als Substituenten diejenigen Reste in Frage kommen, die im Rahmen der Aufzählung der bevorzugten Definitionen genannt wurden.

Die zuvor genannten Reste-Definitionen können untereinander in beliebiger Weise kombiniert werden. Außerdem können auch einzelne Definitionen entfallen.

Verwendet man 3-Cyano-5-chlor-6-(2-chlor-4-fluor-phenyl)-7-(3,3-dimethyl-but-2-yl-amino)-pyrazolo[1,5-a]pyrimidin als Ausgangsstoff und verdünnte Schwefelsäure als Reaktionskomponente, so kann der Verlauf des erfindungsgemäßen Verfahrens (a, Variante α) durch das folgende Formelschema veranschaulicht werden.

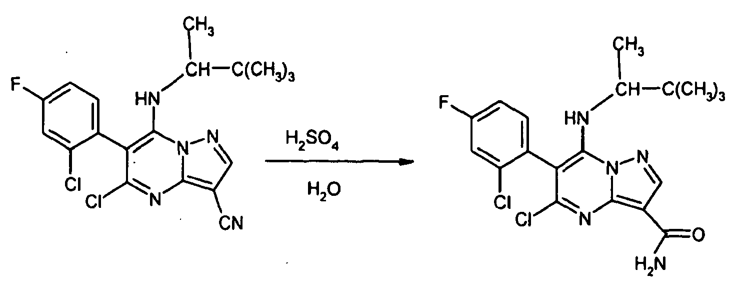

Verwendet man 3-Cyano-5-chlor-6-(2-chlor-4-fluor-phenyl)-7-(3,3-dimethyl-but-2-yl-amino)-pyrazolo[1,5-a]pyrimidin als Ausgangsstoff und Hydroxylammoniumchlorid als Reaktionskomponente, so kann der Verlauf des erfindungsgemäßen Verfahrens (a, Variante β) durch das folgende Formelschema veranschaulicht werden.

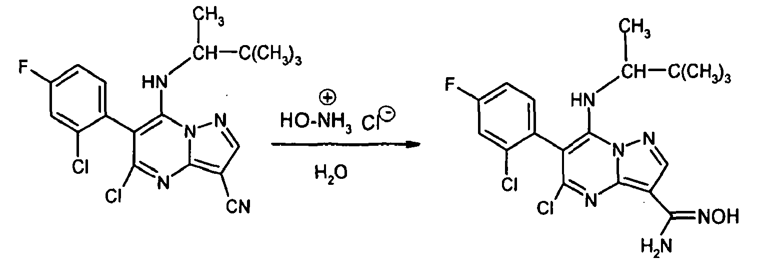

Verwendet man 3-Cyano-5-chlor-6-(2-chlor-4-fluor-phenyl)-7-(3,3-dimethyl-but-2-yl-amino)-pyrazolo[1,5-a]pyrimidin als Ausgangsstoff, Ammoniumchlorid als Reaktionskomponente und Nätrium-methylat als Base, so kann der Verlauf des erfindungsgemäßen Verfahrens (a, Variante γ) durch das folgende Formelschema veranschaulicht werden:

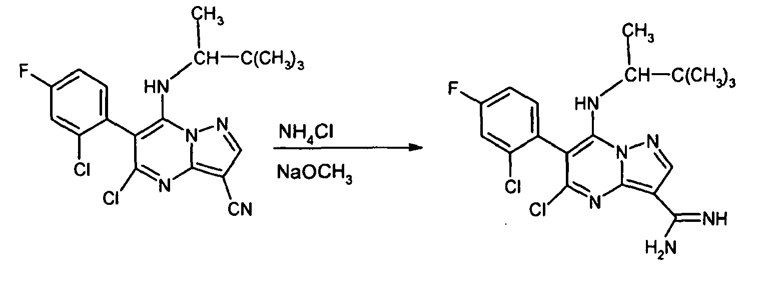

Verwendet man 3-Formyl-5-chlor-6-(2-chlor-4-fluor-phenyl)-7-(3,3-dimethyl-but-2-yl-amino)-pyrazolo [1,5-a]pyrimidin als Ausgangsstoff und 2,4-Dinitro-phenyl-hydrazin als Reaktionskomponente, so kann der Verlauf des erfindungsgemäßen Verfahrens (b) durch das folgende Formelschema veranschaulicht werden.

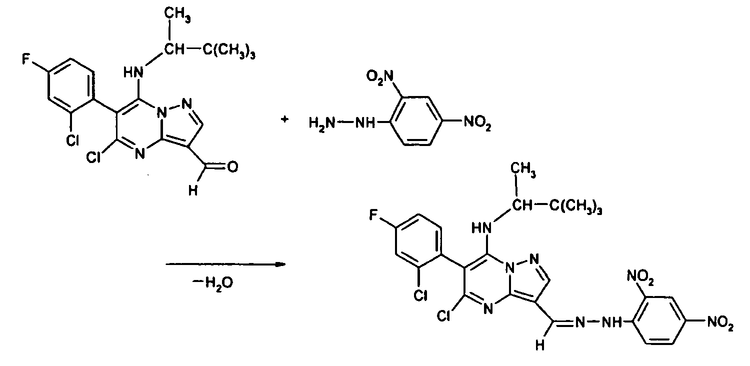

Die bei der Durchführung des erfindungsgemäßen Verfahrens (a) als Ausgangsstoffe benötigten Cyano-Verbindungen sind durch die Formel (II) allgemein definiert. In dieser Formel haben R¹, R², R³, R⁵ und R⁶ vorzugsweise diejenigen Bedeutungen, die bereits im Zusammenhang mit der Beschreibung der erfindungsgemäßen Verbindungen der Formel (I) für diese Reste als bevorzugt genannt wurden.

Die Cyano-Verbindungen der Formel (II) lassen sich herstellen, indem man

### c) Halogen-pyrazolopyrimidine der Formel

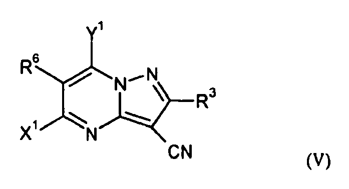

in welcher
- R³ und R⁶: die oben angegebenen Bedeutungen haben,
- X¹: für Halogen steht und
- Y¹: für Halogen steht,
mit Aminen der Formel 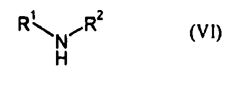 in welcher
- R¹ und R²: die oben angegebenen Bedeutungen haben,
gegebenenfalls in Gegenwart eines Verdünnungsmittels, gegebenenfalls in Gegenwart eines Katalysators und gegebenenfalls in Gegenwart eines Säureakzeptors, umsetzt, und gegebenenfalls in einem zweiten Schritt die so erhaltenen Cyano-Verbindungen der Formel 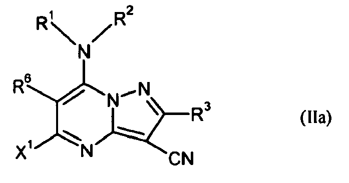 in welcher
- R¹, R², R³,R⁶ und X¹: die oben angegebene Bedeutung haben,
mit Verbindungen der Formel

R⁹-Me (VII)

in welcher
- R⁹: gegebenenfalls substituiertes Alkoxy, gegebenenfalls substituiertes Alkylthio, und
- Me: für Natrium oder Kalium steht,
gegebenenfalls in Gegenwart eines Verdünnungsmittels umsetzt.

Die Halogen-pyrazolopyrimidine der Formel (V) sind bekannt oder lassen sich nach bekannten Methoden herstellen (vgl. DE-A 103 28 996 und PCT/EP 03/05 159).

So erhält man Halogen-pyrazolopyrimidine der Formel (V), indem man

### d) Dihydroxy-pyrazolopyrimidine der Formel

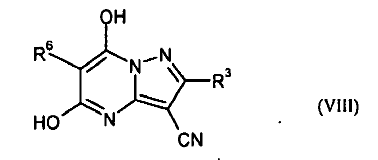

in welcher
- R³ und R⁶: die oben angegebenen Bedeutungen haben,
mit Halogenierungsmitteln gegebenenfalls in Gegenwart eines Verdünnungsmittels umsetzt.

Die Dihydroxy-pyrazolopyrimidine der Formel (VIII) lassen sich herstellen, indem man

### e) Aryl-malonester der Formel

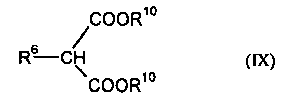

in welcher
- R⁶: die oben angegebenen Bedeutungen hat und
- R¹⁰: für Alkyl steht,
mit Aminopyrazolen der Formel 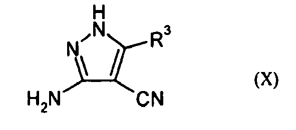 in welcher
- R³: die oben angegebenen Bedeutungen hat;
gegebenenfalls in Gegenwart eines Verdünnungsmittels und gegebenenfalls in Gegenwart einer starken Base umsetzt.

Die bei der Durchführung des Verfahrens (e) als Ausgangsstoffe benötigte Aryl-malonester sind durch die Formel (IX) allgemein definiert. In dieser Formel hat R⁶ vorzugsweise diejenigen Bedeutungen, die bereits im Zusammenhang mit der Beschreibung der erfindungsgemäßen Stoffe der Formel (I) für diesen Rest als bevorzugt genannt wurden. R¹⁰ steht vorzugsweise für Alkyl mit I bis 4 Kohlenstoffatomen, besonders bevorzugt für Methyl oder Ethyl.

Die Aryl-malonester der Formel (IX) sind bekannt oder lassen sich nach bekannten Methoden herstellen (vgl. US-A 6 156 925).

Die bei der Durchführung des Verfahrens (e) als Reaktionskomponenten benötigten Aminopyrazole sind durch die Formel (X) allgemein definiert. In dieser Formel hat R³ vorzugsweise diejenigen Bedeutungen, die bereits im Zusammenhang mit der Beschreibung der erfindungsgemäßen Stoffe der Formel (I) für diesen Rest als bevorzugt genannt wurden.

Als Verdünnungsmittel kommen bei der Durchführung des Verfahrens (e) alle üblichen, inerten organischen Solventien in Betracht. Vorzugsweise verwendbar sind aliphatische, alicyclische oder aromatische Kohlenwasserstoffe, wie Petrolether, Hexan, Heptan, Cyclohexan, Methylcyclohexan, Benzol, Toluol, Xylol oder Decalin; halogenierte Kohlenwasserstoffe, wie Chlorbenzol, Dichlorbenzol, Dichlormethan, Chloroform, Tetrachlormethan, Dichlorethan oder Trichlorethan; Ether, wie Diethylether, Diisopropylether, Methyl+butylether, Methyl-t-amylether, Dioxan, Tetrahydrofuran, 1,2-Dimethoxyethan, 1,2-Diethoxyethan oder Anisol; Nitrile, wie Acetonitril, Propionitril, n- oder i-Butyronitril oder Benzonitril; Amide, wie N,N-Dimethylformamid, N,N-Dimethylacetamid, N-Methylformanilid, N-Methylpyrrolidon oder Hexamethylphosphorsäuretriamid; Ester wie Essigsäuremethylester oder Essigsäureethylester; Sulfoxide, wie Dimethylsulfoxid; Sulfone, wie Sulfolan; Alkohole, wie Methanol, Ethanol, n- oder i-Propanol, n-, i-, sek- oder tert-Butanol, Ethandiol, Propan-1,2-diol, Ethoxyethanol, Methoxyethanol, Diethylenglykolmonomethylether, Diethylenglykolmonoethylether; Amine, wie Tri-n-butylamin oder Carbonsäuren, wie Essigsäure.

Als starke Basen kommen bei der Durchführung des Verfahrens (e) vorzugsweise Erdalkalimetall- oder Alkalimetall-hydride oder -alkoholate sowie Alkalimetallamide in Frage. Beispielhaft genannt seien Natriumhydrid, Natriumamid, Natrium-methylat, Natrium-ethylat und Kalium-tert.-butylat.

Bei der Durchführung des Verfahrens (e) sowie auch bei der Durchführung der anderen in dieser Patentanmeldung beschriebenen Verfahren arbeitet man im Allgemeinen unter Atmosphärendruck.

Es ist aber auch möglich, unter erhöhtem Druck oder, -sofern keine leicht flüchtigen Reaktionskomponenten enthalten sind-, unter vermindertem Druck zu arbeiten.

Die Reaktionstemperaturen können bei der Durchführung des Verfahrens (e) jeweils innerhalb eines größeren Bereiches variiert werden. Bei Abwesenheit von Basen arbeitet man im Allgemeinen bei Temperaturen zwischen 100°C und 250°C, vorzugsweise zwischen 120°C und 200°C. Bei Anwesenheit von Basen arbeitet man im Allgemeinen bei Temperaturen zwischen 20°C und 120°C, vorzugsweise zwischen 20°C und 80°C.

Bei der Durchführung des Verfahrens (e) setzt man auf 1 Mol an Aryl-malonester der Formel (IX) im Allgemeinen 1 bis 15 Mol, vorzugsweise 1 bis 8 Mol an Aminopyrazol der Formel (X) ein. Die Aufarbeitung erfolgt nach üblichen Methoden.

Als Halogenierungsmittel kommen bei der Durchführung des Verfahrens (d) alle üblichen Reagenzien in Betracht, die für einen Austausch von an Kohlenstoff gebundene Hydroxygruppen gegen Halogen geeignet sind. Vorzugsweise verwendbar sind Phosphortrichlorid, Phosphortribromid, Phosphorpentachlorid, Phosphoroxychlorid, Phosgen, Thionylchlorid, Thionylbromid oder deren Gemische. Die entsprechenden Fluor-Verbindungen der Formel (V) lassen sich aus den Chlor- oder Brom-Verbindungen durch Umsetzung mit-Kaliumfluorid herstellen.

Als Verdünnungsmittel kommen bei der Durchführung des Verfahrens (d) alle für derartige Halogenierungen üblichen organischen Solventien in Frage. Vorzugsweise verwendbar sind aliphatische, alicyclische oder aromatische Kohlenwasserstoffe, wie Petrolether, Hexan, Heptan, Cyclohexan, Methylcyclohexan, Benzol, Toluol, Xylol oder Decalin; halogenierte Kohlenwasserstoffe, wie Chlorbenzol, Dichlorbenzol, Dichlormethan, Chloroform, Tetrachlormethan, Dichlorethan oder Trichlorethan.

Als Verdünnungsmittel kann aber auch das Halogenierungsmittel selbst oder ein Gemisch aus Halogenierungsmittel und einem der genannten Verdünnungsmittel dienen.

Die Reaktionstemperaturen können bei der Durchführung des Verfahrens (d) innerhalb eines größeren Bereiches variiert werden. Im Allgemeinen arbeitet man bei Temperaturen zwischen 20°C und 150°C, vorzugsweise zwischen 40°C und 120°C.

Bei der Durchführung des Verfahrens (d) setzt man auf 1 Mol an Dihydroxy-pyrazolopyrimidin der Formel (VIII) jeweils einen Überschuss an Halogenierungsmittel ein. Die Aufarbeitung erfolgt nach üblichen Methoden.

Die bei der Durchführung des Verfahrens (c) als Ausgangsstoffe benötigten Halogen-pyrazolopyrimidine sind durch die Formel (V) allgemein definiert. In dieser Formel haben R³ und R⁶ vorzugsweise diejenigen Bedeutungen, die bereits in Zusammenhang mit der Beschreibung der erfindungsgemäßen Stoffe der Formel (I) für diese Reste als bevorzugt genannt wurden. X¹ und

Y¹ stehen jeweils vorzugsweise für Fluor, Chlor oder Brom, besonders bevorzugt für Fluor oder Chlor.

Die bei der Durchführung des Verfahrens (c) als Reaktionskomponenten benötigten Amine sind durch die Formel (V1) allgemein definiert. In dieser Formel haben R¹ und R² vorzugsweise diejenigen Bedeutungen, die bereits im Zusammenhang mit der Beschreibung der erfindungsgemäßen Stoffe der Formel (I) für diese Reste als bevorzugt genannt wurden.

Die im zweiten Schritt des Verfahrens (c) als Reaktionskomponenten benötigten Verbindungen sind durch die Formel (VII) allgemein definiert.

bevorzugt sind Verbindungen der Formel (VII), in denen R⁹ für Methoxy, Ethoxy, Methylthio, Methylsulfinyl oder Methylsulfonyl steht und Me für Natrium oder Kalium steht.

Die Amine der Formel (VI) und auch die Verbindungen der Formel (VII) sind bekannt oder lassen sich nach bekannten Methoden herstellen.

Als Verdünnungsmittel kommen bei der Durchführung der ersten Stufe des Verfahrens (c) alle üblichen inerten organischen Solventien in Betracht. Vorzugsweise verwendbar sind halogenierte Kohlenwasserstoffe, wie beispielsweise Chlorbenzol, Dichlorbenzol, Dichlormethan, Chloroform, Tetrachlormethan, Dichlorethan oder Trichlorethan; Ether, wie Diethylether, Diisopropylether, Methyl-t-butylether, Methyl-t-amylether, Dioxan, Tetrahydrofuran, 1,2-Dimethoxyethan, 1,2-Diethoxyethan oder Anisol; Nitrile, wie Acetonitril, Propionitril, n- oder i-Butyronitril oder Benzonitril; Amide, wie N,N-Dimethylformamid, N,N-Dimethylacetamid, N-Methylformanilid, N-Methylpyrrolidon oder Hexamethylphosphorsäuretriamid; Ester wie Essigsäuremethylester oder Essigsäureethylester; Sulfoxide, wie Dimethylsulfoxid; Sulfone, wie Sulfolan.

Als Säureakzeptoren kommen bei der Durchführung der ersten Stufe des Verfahren (c) alle für derartige Umsetzungen üblichen anorganischen oder organischen Basen in Frage. Vorzugsweise verwendbar sind Erdalkalimetall- oder Alkalimetallhydride, -hydroxide, -amide, -alkoholate, -acetate, -carbonate oder -hydrogencarbonate, wie beispielsweise Natriumhydrid, Natriumamid, Lithium-diisopropylamid, Natrium-methylat, Natrium-ethylat, Kalium-tert.-butylat, Natriumhydroxid, Kaliumhydroxid, Natriumacetat, Kaliumacetat, Calciumacetat, Natriumcarbonat, Kaliumcarbonat, Kaliumhydrogencarbonat und Natriumhydrogencarbonat, und außerdem AmmoniumVerbindungen wie Ammoniumhydroxid, Ammoniumacetat und Ammoniumcarbonat, sowie tertiäre Amine, wie Trimethylamin, Triethylamin, Tributylamin, N,N-Dimethylanilin, N,N-Dimethylbenzylamin, Pyridin, N-Methylpiperidin, N-Methylmorpholin, N,N-Dimethylaminopyridin, Diazäbicyclooctan (DABCO), Diazabicyclononen (DBN) oder Diazabicycloundecen (DBU).

Als Katalysatoren kommen bei der Durchführung der ersten Stufe des Verfahrens(c) alle für derartige Umsetzungen üblichen Reaktionsbeschleuniger in Betracht.

Vorzugsweise verwendbar sind Fluoride wie Natriumfluorid, Kaliumfluorid oder Ammoniumfluorid.

Die Reaktionstemperaturen können bei der Durchführung der ersten Stufe des Verfahrens (c) in einem größeren Bereich variiert werden. Im Allgemeinen arbeitet man bei Temperaturen zwischen 0°C und 150°C, vorzugsweise bei Temperaturen zwischen 0°C und 80°C.

Bei der Durchführung der ersten Stufe des Verfahrens (c) setzt man auf 1 mol an Halogenpyrazolopyrimidin der Formel (V) im Allgemeinen 0,5 bis 10 mol, vorzugsweise 0,8 bis 2 mol an Amin der Formel (V1) ein. Die Aufarbeitung erfolgt nach üblichen Methoden.

Bei der Durchführung der zweiten Stufe des Verfahrens (c) kommen als Verdünnungsmittel alle üblichen, inerten organischen Solventien in Betracht. Vorzugsweise verwendbar sind halogenierte Kohlenwasserstoffe, wie beispielsweise Chlorbenzol, Dichlorbenzol, Dichlormethan, Chloroform, Tetrachlormethan, Dichlorethan oder Trichlorethan; Ether, wie Diethylether, Diisopropylether, Methyl-t-butylether, Methyl-t-amylether, Dioxan, Tetrahydrofuran, 1,2-Dimethoxyethan, 1,2-Diethoxyethan oder Anisol; Nitrile, wie Acetonitril, Propionitril, n- oder i-Butyronitril oder Benzonitril; Amide, wie N,N-Dimethylformamid, N,N-Dimethylacetamid, N-Methylformanilid, N-Methylpyrrolidon oder Hexamethylphosphorsäuretriamid; Ester wie Essigsäuremethylester oder Essigsäureethylester; Sulfoxide, wie Dimethylsulfoxid; Sulfone, wie Sulfolan.

Auch bei der Durchführung der zweiten Stufe des Verfahrens (c) können die Reaktionstemperaturen in einem größeren Bereich variiert werden. Im Allgemeinen arbeitet man bei Temperaturen zwischen 0°C und 150°C, vorzugsweise zwischen 20°C und 100°C.

Bei der Durchführung der zweiten Stufe des Verfahfrens (c) setzt man die jeweilige Cyano-Verbindung der Formel (IIa) mit einer äquivalenten Menge oder mit einem Überschuß an einer Verbindung der Formel (VII) um. Die Aufarbeitung erfolgt nach üblichen Methoden.

Als Säuren kommen bei der Durchführung des erfindungsgemäßen Verfahrens (a, Variante α) alle üblichen Säuren in Betracht, die zur Verseifung von Nitrilen geeignet sind. Vorzugsweise verwendbar sind anorganische Säuren, wie Salzsäure oder Schwefelsäure.

Als Verdünnungsmittel kommen bei der Durchführung des erfindungsgemäßen Verfahrens (a, Variante α) übliche, inerte organische Solventien in Frage. Beispielhaft genannt seien Ether, wie Diethylether, Tetrahydrofuran oder Dioxan. Außerdem kann Wasser sowohl als Reaktionskomponente als auch als Verdünnungsmittel eingesetzt werden. Besonders bevorzugt verwendet man verdünnte wässrige Säuren, die gleichzeitig als Verdünnungsmittel und als Reaktionskomponente fungieren.

Die Reaktionstemperaturen können bei der Durchführung des erfindungsgemäßen Verfahrens (a, Variante α innerhalb eines bestimmten Bereiches variiert werden. Im Allgemeinen arbeitet man bei Temperaturen zwischen 0°C und 60°C, vorzugsweise zwischen 10°C und 50°C.

Bei der Durchführung des erfindungsgemäßen Verfahrens (a, Variante α) setzt man auf 1 Mol an Cyano-Verbindung der Formel (II) äquivalente Mengen oder auch einen Überschuss an Saure und Wasser ein. Die Aufarbeitung erfolgt nach üblichen Methoden. Im Allgemeinen verfährt man in der Weise, dass man das Reaktionsgemisch, gegebenenfalls nach vorherigem Einengen, mit Eis verrührt und den anfallenden Niederschlag absaugt.

Als Reaktionskomponenten kommen bei der Durchführung des erfindungsgemäßen Verfahrens (a, Variante β) Hydroxylamin oder Hydroxylammonium-Salze, wie Chlorid oder Sulfat in Betracht. Vorzugsweise verwendbar ist Hydroxylammoniumchlorid.

Als Verdünnungsmittel kommen bei der Durchführung des erfindungsgemäßen Verfahrens (a, Variante β) alle üblichen, inerten organischen Solventien in Frage. Vorzugsweise verwendbar sind Alkohole, wie Methanol, Ethanol, n-Propanol oder Isopropanol.

Als Katalysatoren kommen bei der Durchführung des erfindungsgemäßen Verfahrens (a, Variante β) alle für derartige Umsetzungen üblichen Reaktionsbeschleuniger in Betracht. Vorzugsweise verwendbar sind saure oder basische Katalysatoren, wie zum Beispiel der unter der Bezeichnung Amberlyst A-21^{®} im Handel befindliche, schwach basische Ionenaustauscher.

Die Reaktionstemperaturen können bei der Durchführung des erfindungsgemäßen Verfahrens (a, Variante β) innerhalb eines bestimmten Bereiches variiert werden. Im Allgemeinen arbeitet man bei Temperaturen zwischen 0° und 80°C, vorzugsweise zwischen 10°C und 60°C.

Bei der Durchführung des erfindungsgemäßen Verfahrens (a, Variante β) setzt man auf 1 Mol an Cyano-Verbindung der Formel (II) im Allgemeinen eine äquivalente Menge oder auch einen Überschuss, vorzugsweise zwischen 1,1 und 1,5 Mol an Hydroxylamin oder Hydroxylammonium-Salz ein. Die Aufarbeitung erfolgt nach üblichen Methoden. Im Allgemeinen geht man so vor, dass man das Reaktionsgemisch gegebenenfalls filtriert, dann einengt und das isolierte Produkt reinigt.

Als Basen kommen bei der Durchführung des erfindungsgemäßen Verfahrens (a, Variante γ), alle für derartige Reaktionen üblichen, organischen Basen in Betracht. Vorzugsweise verwendbar sind Alkalimetallalkoholate, wie Natriuin-methylat oder Kalium-tert-butylat.

Als Verdünnungsmittel kommen bei der Durchführung des erfindungsgemäßen Verfahrens (a, Variante γ) alle für derartige Reaktionen üblichen, organischen Solventien in Frage. Vorzugsweise verwendbar sind Alkohole, wie Methanol, Ethanol, n-Propanol oder Isopropanol.

Die Reaktionstemperaturen können auch bei der Durchführung des erfindungsgemäßen Verfahrens (a, Variante γ) innerhalb eines bestimmten Bereiches variiert werden. Im Allgemeinen arbeitet man bei Temperaturen zwischen 0°C und 80°C, vorzugsweise zwischen 10°C und 60°C.

Bei der Durchführung des erfindungsgemäßen Verfahrens (a, Variante γ) setzt man auf 1 Mol an Cyano-Verbindung der Formel (II) im Allgemeinen eine äquivalente Menge oder einen Überschuss an Ammoniumchlorid sowie eine katalytische Meng an Base ein. Die Aufarbeitung erfolgt wiederum nach üblichen Methoden. Die auf diese Weise hergestellten Pyrazolopyrimidine der Formel (I) können dabei auch in Form ihrer Chlorwasserstoff-Additions-Salze isoliert werden.

Die bei der Durchführung des erfindungsgemäßen Verfahrens (b) als Ausgangsstoffe benötigten Carbonyl-Verbindungen sind durch die Formel (III) allgemein definiert. In dieser Formel haben R¹, R², R³, R⁵, R⁷ vorzugsweise diejenigen Bedeutungen, die bereits im Zusammenhang mit der Beschreibung der erfindungsgemäßen Stoffe der Formel (I) für diese Reste als bevorzugt genannt wurden.

Die Carbonyl-Verbindungen der Formel (III) lassen sich herstellen, indem man

### f) Cyano-Verbindungen derFormel

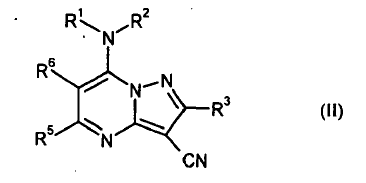

in welcher
R¹, R², R³, R⁵ und R⁶ die oben angegebenen Bedeutungen haben,
entweder
- α): mit Düsobutyl-aluminiumhydrid in Gegenwart von wässriger Ammoniumchlorid-Lösung sowie in Gegenwart eines organischen Verdünnungsmittels umsetzt,
oder
- β): mit Grignard-Verbindungen der Formel

R¹¹-Mg-X² (XI)

in welcher
R¹¹ für Alkyl steht und
X² für Chlor, Brom oder Iod steht, in Gegenwart eines Verdünnungsmittels und gegebenenfalls in Gegenwart eines Katalysators umsetzt,
oder

### g) Pyrazolopyrimidine der Formel

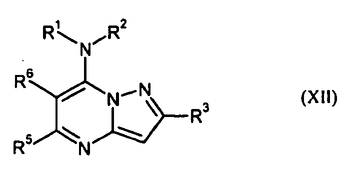

in welcher
R¹, R², R³, R⁵ und R⁶ die oben angegebenen Bedeutungen haben,
mit Säurehalogeniden der Formel 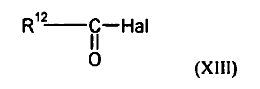 in welcher
- R¹²: für Alkyl steht und
- Hal: für Chlor oder Brom steht,
oder
mit Säureanhydriden der Formel 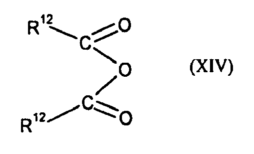 in welcher
- R¹²: für Alkyl steht,
jeweils in Gegenwart eines Katalysators und in Gegenwart eines Verdünnungsmittels umsetzt,
oder

### h) Hydroxy-pyrazolopyrimidine der Formel

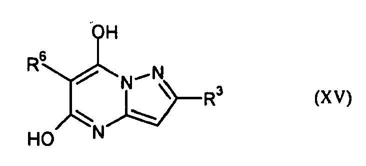

in welcher
R³ und R⁶ die oben angegebenen Bedeutungen haben,
mit Phosphoroxychlorid in Gegenwart von Dimethylformamid umsetzt und gegebenenfalls unter Zugabe von Phosphorpentachlorid nachreagieren lässt, und die dabei entstehenden Halogeno-pyrazolopyrimidine der Formel 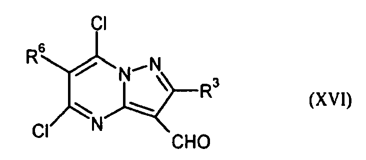 in welcher
R³ und R⁶ die oben angegebenen Bedeutungen haben,
mit Aminen der Formel 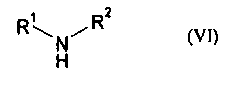 in welcher
R¹ und R² die oben angegebenen Bedeutungen haben,
gegebenenfalls in Gegenwart eines Katalysators, gegebenenfalls in Gegenwart eines Säurebindemittels und gegebenenfalls in Gegenwart eines Verdünnungsmittels umsetzt.

Die bei der Durchführung des Verfahrens (f, Variante β) als Reaktionskomponenten benötigten Grignard-Verbindungen sind durch die Formel (XI) allgemein definiert. In dieser Formel steht R¹ vorzugsweise für Alkyl mit 1 bis 4 Kohlenstoffatomen, besonders bevorzugt für Methyl oder Ethyl. X² steht auch vorzugsweise für Chlor, Brom oder Iod.

Als Verdünnungsmittel kommen bei der Durchführung des Verfahrens (f, Variante α), alle üblichen inerten, organischen Solventien in Frage. Vorzugsweise verwendbar sind aliphatische oder aromatische, gegebenenfalls halogenierte Kohlenwasserstoffe, wie Toluol, Dichlormethan, Chloroform oder Tetrachlorkohlenstoff.

Die Reaktionstemperaturen können bei der Durchführung des Verfahrens (f, Variante α) innerhalb eines bestimmten Bereiches variiert werden. Im Allgemeinen arbeitet man bei Temperaturen zwischen -80°C und +20°C, vorzugsweise zwischen -60°C und +10°C.

Bei der Durchführung des Verfahrens (f, Variante α) setzt man auf 1 Mol an Cyano-Verbindung der Formel (II) im Allgemeinen eine äquivalente Menge oder auch einen Überschuss, vorzugsweise 1, 1 bis 1,2 Mol an Di-isobutylaluminium-hydrid ein und fügt anschließend einen Überschuss an wässriger Ammoniumchlorid-Lösung hinzu. Die Aufarbeitung erfolgt nach üblichen Methoden. Im Allgemeinen verfährt man in der Weise, dass man das Reaktionsgemisch ansäuert, die organische Phase abtrennt, die wässrige Phase mit einem mit Wasser wenig mischbaren organischen Solvens extrahiert, die vereinigten organischen Phasen wäscht, trocknet und unter vermindertem Druck einengt.

Als Katalysatoren kommen bei der Durchführung des Verfahrens (f, Variante β) alle für Grignard-Reaktionen üblichen Reaktionsbeschleuniger in Betracht. Beispielsweise genannt seien Kaliumiodid und Iod.

Als Verdünnungsmittel kommen bei der Durchführung des Verfahrens (f, Variante β) alle für derartige Umsetzungen üblichen, inerten organischen Solventien in Frage. Vorzugsweise verwendbar sind Ether, wie Diethylether, Dioxan oder Tetrahydrofuran, außerdem aromatische Kohlenwasserstoffe, wie Toluol, und auch Gemische aus Ethern und aromatischen Kohlenwasserstoffen, wie Toluol/tetrahydofuran.

Die Reaktionstemperaturen können bei der Durchführung desVerfahrens (f, Variante β) in einem bestimmten Bereich variiert werden. Im Allgemeinen arbeitet man bei Temperaturen zwischen -20°C und + 100°C, vorzugsweise zwischen 0°C und 80°C.

Bei der Durchführung des Verfahrens (f, Variante β) setzt man auf I Mol an Cyano-Verbindung der Formel (II) im Allgemeinen 2 bis 3 Mol an Grignard-Verbindung der Formel (XI) ein. Anschließend erfolgt eine wässrige Aufarbeitung nach üblichen Methoden.

Die bei der Durchführung des Verfahrens (g) als Ausgangsstoffe benötigten Pyrazolo-pyrimidine sind durch die Formel (XII) allgemein definiert. In dieser Formel haben R¹, R², R³, R⁵ und R⁶ vorzugsweise diejenigen Bedeutungen, die bereits im Zusammenhang mit der Beschreibung der erfindungsgemäßen Stoffe der Formel (I) für diese Reste als bevorzugt genannt wurden.

Die Pyrazolopyrimidine der Formel (XII) sind bekant oder lassen sich nach bekannten Methoden herstellen.

Die bei der Durchführung des Verfahrens (d) als Reaktionskomponenten benötigten Säurehalogenide und Säureanhydride sind durch die Formeln (XIII) und (XIV) allgemein definiert. In diesen Formeln steht R¹² vorzugsweise für Alkyl mit 1 bis 4 Kohlenstoffatomen, besonders bevorzugt für Methyl, Ethyl oder Propyl. Hal steht in der Formel (XIII) bevorzugt für Chlor oder Brom.

Sowohl die Säurehalogenide der Formel (XIII) als auch die Säureanhydride der Formel (XIV) sind bekannt oder lassen sich nach bekannten Methoden herstellen.

Als Katalysatoren kommen bei der Durchführung des Verfahrens (g) alle für Friedel-Crafts-Reaktionen Oblicherweise verwendbaren Reaktionsbeschleuniger in Betracht. Vorzugsweise verwendbar sind Lewis-Säuren, wie Aluminium-trichlorid, Aluminium-tribromid und Eisen(III)chlorid.

Als Verdünnungsmittel kommen bei der Durchführung des Verfahrens (g) alle für derartige Friedel-Crafts-Reaktionen üblichen, inerten organischen Solventien in Frage. Vorzugsweise verwendbar sind Ether, wie Diethylether, Methyl-tert-bugylether, Dioxan und Tetrahydrofuran, sowie auch Schwefelkohlenstoff.

Die Reaktionstemperaturen können bei der Durchführung des Verfahrens (g) in einem bestimmten Bereich variiert werden. Im Allgemeinen arbeitet man bei Temperaturen zwischen -10°C und +100°C, vorzugsweise zwischen 0°C und 80°C.

Bei der Durchführung des Verfahrens (g) setzt man auf 1 mol an Pyrazolo-pyrimidin der Formel (XII) im Allgemeinen 1 bis 5 mol, vorzugsweise 1 bis 2 mol an Säurehalogenid der Formel (XIII) und 1,1 bis 5 mol, vorzugsweise 1,1 bis 3 mol an Katalysator, beziehungsweise 1 bis 5 mol, vorzugsweise 1 bis 2 mol an Säureanhydrid der Formel (XIV) und 2,1 bis 6 mol, vorzugsweise 2,1 bis 4 mol an Katalysator ein. Man verfährt im Allgemeinen in der Weise, dass man die Reaktionskomponenten zunächst bei niedriger Temperatur zusammengibt und nach dem Abklingen der anfangs heftigen Reaktion allmählich bis auf Rückflusstemperatur erhitzt. Die Aufarbeitung erfolgt nach üblichen Methoden.

Die bei der Durchführung des Verfahrens (h) als Ausgangsstoffe benötigten Hydroxy-pyrazolopyrimidine sind durch die Formel (XV) allgemein definiert. In dieser Formel haben R³ und R⁶ vorzugsweise diejenigen Bedeutungen, die bereits im Zusammenhang mit der Beschreibung der erfindungsgemäßen Stoffe der Formel (I) für diese Reste als bevorzugt genannt wurden.

Die Hydroxy-pyrazolopyrimidine der Formel (XV) lassen sich nach dem Verfahren (e) herstellen, wenn man Aminopyrazole der Formel (X) einsetzt, die statt der CN-Gruppe ein Wasserstoffatom tragen.

Die erste Stufe des Verfahrens (h) wird unter den Bedingungen des Vilsmeier-Formylierungen mit Hilfe von Phosphoroxychlorid in Gegenwart von Dimethylformamid durchgeführt. Dabei kann auch Phosphorpentachlorid als Chlorierungsmittel hinzugefügt werden.

Die Reaktionstemperaturen können bei der Durchführung der ersten Stufe des Verfahrens (h) in einem größeren Bereich variiert werden. Im Allgemeinen arbeitet man bei Temperaturen zwischen -10°C und +150°C, vorzugsweise zwischen 0°C und 120°C.

Bei der Durchführung der ersten Stufe des Verfahrens (h) setzt man auf 1 mol an Hydroxypyrazolopyrimidin der Formel (XV) im Allgemeinen 2 bis 5 mol an Dimethylformamid, 5 bis 15 mol Phosphoroxychlorid und gegebenenfalls 0 bis 2 mol Phosphorpentachlorid ein. Die Aufarbeitung erfolgt nach üblichen Methoden.

Bei der Durchführung der zweiten Stufe des Verfahrens (h) kommen die Amine der Formel (VI) sowie diejenigen Katalysatoren, Säurebindemittel und Verdünnungsmittel in Betracht, die bereits im Zusammenhang mit der Beschreibung der ersten Stufe des Verfahrens (c) genannt wurden. Auch die Reaktionstemperaturen und die übrigen Umsetzungsbedingungen entsprechen denjenigen, die im Falle der ersten Stufe des Verfahrens (c) angewandt werden.

Die bei der Durchführung des erfindungsgemäßen Verfahrens (b) als Reaktionskomponenten benötigten Amino-Verbindungen sind durch die Formel (IV) allgemein definiert. In dieser Formel hat R⁸ vorzugsweise diejenigen Bedeutungen, die bereits im Zusammenhang mit der Beschreibung der erfindungsgemäßen Stoffe der Formel (I) für diesen Rest als bevorzugt genannt wurden. Die Amino-Verbindungen der Formel (IV) können auch in Form ihrer Säureadditions-Salze eingesetzt werden. Bevorzugt in Frage kommen dabei die Salze, die durch Addition von Chlorwasserstoff oder Schwefelsäure entstehen.

Sowohl die Amino-Verbindungen der Formel (IV) als auch deren Säureadditions-Salze sind bekannt oder lassen sich nach bekannten Methoden herstellen.

Als Verdünnungsmittel kommen bei der Durchführung des erfindungsgemäßen Verfahrens (b) alle üblichen, inerten organischen Solventien in Frage. Vorzugsweise verwendbar sind Alkohole, wie Methanol, Ethanol, n-Propanol oder Isopropanol, außerdem Kohlenwasserstoffe, wie Benzol oder Toluol.

Als Katalysatoren kommen bei der Durchführung des erfindungsgemäßen Verfahrens (b) alle für derartige Umsetzungen üblichen Reaktionsbeschleuniger in Betracht. Vorzugsweise verwendbar sind saure Katalysatoren, wie Schwefelsäure oder p-Toluolsulfonsäure, oder basische Katalysatoren, wie zum Beispiel der unter der Bezeichnung Amberlyst A-21^{®} im Handel befindliche, schwach basische Ionenaustauscher.

Die Reaktionstemperaturen können bei der Durchführung des erfindungsgemäßen Verfahrens (b) innerhalb eines größeren Bereiches variiert werden. Im Allgemeinen arbeitet man bei Temperaturen zwischen 0°C und 150°C, vorzugsweise zwischen 10°C und 130°C.

Bei der Durchführung des erfindungsgemäßen Verfahrens (b) setzt man auf 1 Mol an CarbonylVerbindung der Formel (III) im Allgemeinen eine äquivalente Menge oder einen Überschuss, vorzugsweise zwischen 1,0 und 1,5 Mol an Amino-Verbindung der Formel (IV) oder eines Säureadditions-Salzes davon ein. Die Aufarbeitung erfolgt wiederum nach üblichen Methoden.

Die erfindungsgemäßen Stoffe weisen eine starke mikrobizide Wirkung auf und können zur Bekämpfung von unerwünschten Mikroorganismen, wie Fungi und Bakterien, im Pflanzenschutz und im Materialschutz eingesetzt werden.

Fungizide lassen sich Pflanzenschutz zur Bekämpfung von Plasmodiophoromycetes, Oomycetes, Chytridiomycetes, Zygomycetes, Ascomycetes, Basidiomycetes und Deuteromycetes einsetzen.

Bakterizide lassen sich im Pflanzenschutz zur Bekämpfung von Pseudomonadaceae, Rhizobiaceae, Enterobacteriaceae, Corynebacteriaceae und Streptomycetaceae einsetzen.

Beispielhaft aber nicht begrenzend seien einige Erreger von pilzlichen und bakteriellen Erkrankungen, die unter die oben aufgezählten Oberbegriffe fallen, genannt:
Xanthomonas-Arten, wie beispielsweise Xanthomonas campestris pv. oryzae;
Pseudomonas-Arten, wie beispielsweise Pseudomonas syringae pv. lachrymans;
Erwinia-Arten, wie beispielsweise Erwinia amylovora;
Pythium-Arten, wie beispielsweise Pythium ultimum;
Phytophthora-Arten, wie beispielsweise Phytophthora infestans;
Pseudoperonospora-Arten, wie beispielsweise Pseudoperonospora humuli oder
Pseudoperonospora cubensis;
Plasmopara-Arlen, wie beispielsweise Plasmopara viticola;
Bremia-Arten, wie beispielsweise Bremia lactucae;
Peronospora-Arten, wie beispielsweise Peronospora pisi oder P. brassicae;
Erysiphe-Arten, wie beispielsweise Erysiphe graminis;
Sphaerotheca-Arten, wie beispielsweise Sphaerotheca fuliginea;
Podosphaera-Arten, wie beispielsweise Podosphaera leucotricha;
Venturia-Arten, wie beispielsweise Venturia inaequalis;
Pyrenophora-Arten, wie beispielsweise Pyrenophorateres oder P. graminea
(Konidienform: Drechslera, Syn: Helminthosporium);
Cochliobolus-Arten, wie beispielsweise Cochliobolus sativus
(Konidienform: Drechslera, Syn: Helminthosporium);
Uromyces-Arten, wie beispielsweise Uromyces appendiculatus;
Puccinia-Arten, wie beispielsweise Puccinia recondita;
Sclerotinia-Arten, wie beispielsweise Sclerotinia sclerotiorum;
Tilletia-Arten, wie beispielsweise Tilletia caries;
Ustilago-Arten, wie beispielsweise Ustilago nuda oder Ustilago avenae;
Pellicularia-Arten, wie beispielsweise Pellicularia sasakii;
Pyricularia-Arten, wie beispielsweise Pyricularia oryzae;
Fusarium-Arten, wie beispielsweise Fusarium culmorum;
Botrytis-Arten, wie beispielsweise Botrytis cinerea;
Septoria-Arten, wie beispielsweise Septoria nodorum;
Leptosphaeria-Arten, wie beispielsweise Leptosphaeria nodorum;
Cercospora-Arten, wie beispielsweise Cercospora canescens;
Alternaria-Arten, wie beispielsweise Alternaria brassicae;
Pseudocercosporella-Arten, wie beispielsweise Pseudocercosporella herpotrichoides.

Die erfindungsgemäßen Wirkstoffe weisen auch eine sehr gute stärkende Wirkung in Pflanzen auf. Sie eignen sich daher zur Mobilisierung pflanzeneigener Abwehrkräfte gegen Befall durch unerwünschte Mikroorganismen.

Unter pflanzenstärkenden (resistenzinduzierenden) Stoffen sind im vorliegenden Zusammenhang solche Substanzen zu verstehen, die in der Lage sind, das Abwehrsystem von Pflanzen so zu stimulieren, dass die behandelten Pflanzen bei nachfolgender Inokulation mit unerwünschten Mikroorganismen weitgehende Resistenz gegen diese Mikroorganismen entfalten.

Unter unerwünschten Mikroorganismen sind im vorliegenden Fall phytopathogene Pilze, Bakterien und Viren zu verstehen. Die erfindungsgemäßen Stoffe können also eingesetzt werden, um Pflanzen innerhalb eines gewissen Zeitraumes nach der Behandlung gegen den Befall durch die genannten Schaderreger zu schützen. Der Zeitraum, innerhalb dessen Schutz herbeigeführt wird, erstreckt sich im allgemeinen von 1 bis 10 Tage, vorzugsweise 1 bis 7 Tage nach der Behandlung der Pflanzen mit den Wirkstoffen.

Die gute Pflanzenverträglichkeit der Wirkstoffe in den zur Bekämpfung von Pflanzenkrankheiten notwendigen Konzentrationen erlaubt eine Behandlung von oberirdischen Pflanzenteilen, von Pflanz- und Saatgut, und des Bodens.

Dabei lassen sich die erfindungsgemäßen Wirkstoffe mit besonders gutem Erfolg zur Bekämpfung von Getreidekrankheiten, wie beispielsweise gegen Erysiphe-Arten, von Krankheiten im Wein-, Obst- und Gemüseanbau, wie beispielsweise gegen Botrytis-, Venturia-, Sphaerotheca- und Podosphaera-Arten, einsetzen.

Die erfindungsgemäßen Wirkstoffe eignen sich auch zur Steigerung des Emteertrages. Sie sind außerdem mindertoxisch und weisen eine gute Pflanzenverträglichkeit auf.

Die erfindungsgemäßen Wirkstoffe können gegebenenfalls in bestimmten Konzentrationen und Aufwandmengen auch als Herbizide, zur Beeinflussung des Pflanzenwachstums, sowie zur Bekämpfung von tierischen Schädlingen verwendet werden. Sie lassen sich gegebenenfalls auch als Zwischen- und Vorprodukte für die Synthese weiterer Wirkstoffe einsetzen.

Erfindungsgemäß können alle Pflanzen und Pflanzenteile behandelt werden. Unter Pflanzen werden hierbei alle Pflanzen und Pflanzenpopulationen verstanden, wie erwünschte und unerwünschte Wildpflanzen oder Kulturpflanzen (einschließlich natürlich vorkommender Kulturpflanzen). Kulturpflanzen können Pflanzen sein, die durch konventionelle Züchtungs- und Optimierungsmethoden oder durch biotechnologische und gentechnologische Methoden oder Kombinationen dieser Methoden erhalten werden können, einschließlich der transgenen Pflanzen und einschließlich der durch Sortenschutzrechte schützbaren oder nicht schützbaren Pflanzensorten. Unter Pflanzenteilen sollen alle oberirdischen und unterirdischen Teile und Organe der Pflanzen, wie Spross, Blatt, Blüte und Wurzel verstanden werden, wobei beispielhaft Blätter, Nadeln, Stängel, Stämme, Blüten, Fruchtkörper, Früchte und Samen sowie Wurzeln, Knollen und Rhizome aufgeführt werden. Zu den Pflanzenteilen gehört auch Erntegut sowie vegetatives und generatives Vermehrungsmaterial, beispielsweise Stecklinge, Knollen, Rhizome, Ableger und Samen.

Die erfindungsgemäße Behandlung der Pflanzen und Pflanzenteile mit den Wirkstoffen erfolgt direkt oder durch Einwirkung auf deren Umgebung, Lebensraum oder Lagerraum nach den üblichen Behandlungsmethoden, z.B. durch Tauchen, Sprühen, Verdampfen, Vernebeln, Streuen, Aufstreichen und bei Vermehrungsmaterial, insbesondere bei Samen, weiterhin durch ein- oder mehrschichtiges Umhüllen.

Im Materialschutz lassen sich die erfindungsgemäßen Stoffe zum Schutz von technischen Materialien gegen Befall und Zerstörung durch unerwünschte Mikroorganismen einsetzen.

Unter technischen Materialien sind im vorliegenden Zusammenhang nichtlebende Materialien zu verstehen, die für die Verwendung in der Technik zubereitet worden sind. Beispielsweise können technische Materialien, die durch erfindungsgemäße Wirkstoffe vor mikrobieller Veränderung oder Zerstörung geschützt werden sollen, Klebstoffe, Leime, Papier und Karton, Textilien, Leder, Holz, Anstrichmittel und Kunststoffartikel, Kühlschmierstoffe und andere Materialien sein, die von Mikroorganismen befallen oder zersetzt werden können. Im Rahmen der zu schützenden Materialien seien auch Teile von Produktionsanlagen, beispielsweise Kühlwasserkreisläufe, genannt, die durch Vermehrung von Mikroorganismen beeinträchtigt werden können. Im Rahmen der vorliegenden Erfindung seien als technische Materialien vorzugsweise Klebstoffe, Leime, Papiere und Kartone, Leder, Holz, Anstrichmittel, Kühlschmiermittel und Wärmeübertragungsflüssigkeiten genannt, besonders bevorzugt Holz

Als Mikroorganismen, die einen Abbau oder eine Veränderung der technischen Materialien bewirken können, seien beispielsweise Bakterien, Pilze, Hefen, Algen und Schleimorganismen genannt. Vorzugsweise wirken die errndungsgemäßen Wirkstoffe gegen Pilze, insbesondere Schimmelpilze, holzverfärbende und holzzerstörende Pilze (Basidiomyceten) sowie gegen Schleimorganismen und Algen.

Es seien beispielsweise Mikroorganismen der folgenden Gattungen genannt:
Alternaria, wie Alternaria tenuis,
Aspergillus, wie Aspergillus niger,
Chaetomium, wie Chaetomium globosum,
Coniophora, wie Coniophora puetana,
Lentinus, wie Lentinus tigrinus,
Penicillium, wie Penicillium glaucum,
Polyporus, wie Polyporus versicolor,
Aureobasidium, wie Aureobasidium pullulans,
Sclerophoma, wie Sclerophoma pityophila,
Trichoderma, wie Trichoderma viride,
Escherichia, wie Escherichia coli,
Pseudomonas, wie Pseudomonas aeruginosa,
Staphylococcus, wie Staphylococcus aureus.

Die Wirkstoffe können in Abhängigkeit von ihren jeweiligen physikalischen und/ oder chemischen Eigenschaften in die üblichen Formulierungen überführt werden, wie Lösungen, Emulsionen, Suspensionen, Pulver, Schäume, Pasten, Granulate, Aerosole, Feinstverkapselungen in polymeren Stoffen und in Hüllmassen für Saatgut, sowie ULV-Kalt- und Warmnebel-Formulierungen.

Diese Formulierungen werden in bekannter Weise hergestellt, z.B. durch Vermischen der Wirkstoffe mit Streckmitteln, also flüssigen Lösungsmitteln, unter Druck stehenden verflüssigten Gasen und/oder festen Trägerstoffen, gegebenenfalls unter Verwendung von oberflächenaktiven Mitteln, also Emulgiermitteln und/oder Dispergiermitteln und/oder schaumerzeugenden Mitteln. Im Falle der Benutzung von Wasser als Streckmittel können z.B. auch organische Lösungsmittel als Hilfslösungsmittel verwendet werden. Als flüssige Lösungsmittel kommen im Wesentlichen infrage: Aromaten, wie Xylol, Toluol oder Alkylnaphthaline, chlorierte Aromaten oder chlorierte aliphatische Kohlenwasserstoffe, wie Chlorbenzole, Chlorethylene oder Methylenchlorid, aliphatische Kohlenwasserstoffe, wie Cyclohexan oder Paraffine, z.B. Erdölfraktionen, Alkohole, wie Butanol oder Glycol sowie deren Ether und Ester, Ketone, wie Aceton, Methylethylketon, Methylisobutylketon oder Cyclohexanon, stark polare Lösungsmittel, wie Dimethylformamid und Dimethylsulfoxid, sowie Wasser. Mit verflüssigten gasförmigen Streckmitteln oder Trägerstoffen sind solche Flüssigkeiten gemeint, welche bei normaler Temperatur und unter Normaldruck gasförmig sind, z.B. Aerosol-Treibgase, wie Halogenkohlenwasserstoffe sowie Butan, Propan, Stickstoff und Kohlendioxid. Als feste Trägerstoffe kommen infrage: z.B. natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate. Als feste Trägerstoffe für Granulate kommen infrage: z.B. gebrochene und fraktionierte natürliche Gesteine wie Calcit, Bims, Marmor, Sepiolith, Dolomit sowie synthetische Granulate aus anorganischen und organischen Mehlen sowie Granulate aus organischem Material wie Sägemehl, Kokosnussschalen, Maiskolben und Tabakstängel. Als Emulgier und/oder schaumerzeugende Mittel kommen infrage: z.B. nichtionogene und anionische Emulgatoren, wie Polyoxyethylen-Fettsäureester, Polyoxyethylen-Fettalkoholether, z.B. Alkylarylpolyglycolether, Alkylsulfonate, Alkylsulfate, Arylsulfonate sowie Eiweißhydrolysate. Als Dispergiermittel kommen infrage: z.B. Lignin-Sulfitablaugen und Methylcellulose.

Es können in den Formulierungen Haftmittel wie Carboxymethylcellulose, natürliche und synthetische pulverige, körnige oder latexförmige Polymere verwendet werden, wie Gummiarabicum, Polyvinylalkohol, Polyvinylacetat, sowie natürliche Phospholipide, wie Kephaline und Lecithine, und synthetische Phospholipide. Weitere Additive können mineralische und vegetabile Öle sein.

Es können Farbstoffe wie anorganische Pigmente, z.B. Eisenoxid, Titanoxid, Ferrocyanblau und organische Farbstoffe, wie Alizarin-, Azo- und Metallphthalocyaninfarbstoffe und Spurennährstoffe, wie Salze von Eisen, Mangan, Bor, Kupfer, Kobalt, Molybdän und Zink verwendet werden.

Die Formulierungen enthalten im allgemeinen zwischen 0,1 und 95 Gewichtsprozent Wirkstoff; vorzugsweise zwischen 0,5 und 90 %.

Die erfindungsgemäßen Wirkstoffe können als solche oder in ihren Formulierungen auch in Mischung mit bekannten Fungiziden, Bakteriziden, Akariziden, Nematiziden oder Insektiziden verwendet werden, um so z.B. das Wirkungsspektrum zu verbreitern oder Resistenzentwicklungen vorzubeugen. In vielen Fällen erhält man dabei synergistische Effekte, d.h. die Wirksamkeit der Mischung ist größer als die Wirksamkeit der Einzelkomponenten.

Als Mischpartner kommen zum Beispiel folgende Verbindungen in Frage:

### Fungizide:

2-Phenylphenol; 8-Hydroxyquinoline sulfate; Acibenzolar-S-methyl; Aldimorph; Amidoflumet; Ampropylfos; Ampropylfos-potassium; Andoprim; Anilazine; Azaconazole; Azoxystrobin; Benalaxyl; Benalaxyl-M; Benodanil; Benomyl; Benthiavalicarb-isopropyl; Benzamacril; Benzamacril-isobutyl; Bilanafos; Binapacryl; Biphenyl; Bitertanol; Blasticidin-S; Boscalid; Bromuconazole; Bupirimate; Buthiobate; Butylamine; Calcium polysulfide; Capsimycin; Captafol; Captan; Carbendazim; Carboxin; Carpropamid; Carvone; Chinomethionat; Chlobenthiazone; Chlorfenazole; Chloroneb; Chlorothalonil; Chlozolinate; Clozylacon; Cyazofarnid; Cyflufenamid; Cymoxanil; Cyproconazole; Cyprodinil; Cyprofuram; Dagger G; Debacarb; Dichlofluanid; Dichlone; Dichlorophen; Diclocymet; Diclomezine; Dicloran; Diethofencarb; Difenoconazole; Diflumetorim; Dimethirimol; Dimethomorph; Dimoxystrobin; Diniconazole; Diniconazole-M; Dinocap; Diphenylamine; Dipyrithione; Ditalimfos; Dithianon; Dodine; Drazoxolon; Edifenphos; Epoxiconazole; Ethaboxam; Ethirimol; Etridiazole; Famoxadone; Fenamidone; Fenapanil; Fenarimol; Fenbuconazole; Fenfuram; Fenhexamid; Fenitropan; Fenoxanil; Fenpiclonil; Fenpropidin; Fenpropimorph; Ferbam; Fluazinam; Flubenzimine; Fludioxonil; Flumetover, Flumorph; Fluoromide; Fluoxastrobin; Fluquinconazole; Flurprimidol; Flusilazole; Flusulfamide; Flutolanil; Flutriafol; Folpet; Fosetyl-AI; Fosetyl-sodium; Fuberidazole; Furalaxyl; Furametpyr, Furcarbanil; Furmecyclox; Guazatine; Hexachlorobenzene; Hexaconazole; Hymexazol; Imazalil; Imibenconazole; Iminoctadine triacetate; Iminoctadine tris(albesilate); Iodocarb; Ipconazole; Iprobenfos; Iprodione; Iprovalicarb; Irumamycin; Isoprothiolane; Isovaledione; Kasugamycin; Kresoxim-methyl; Mancozeb; Maneb; Meferimzone; Mepanipyrim; Mepronil; Metalaxyl; Metalaxyl-M; Metconazole; Methasulfocarb; Methfuroxam; Metiram; Metominostrobin; Metsulfovax; Mildiomycin; Myclobutanil; Myclozolin; Natamycin; Nicobifen; Nitrothal-isopropyl; Noviflumuron; Nuarimol; Ofurace; Orysastrobin; Oxadixyl; Oxolinic acid; Oxpoconazole; Oxycarboxin; Oxyfenthiin; Paclobutrazol; Pefurazoate; Penconazole; Pencycuron; Phosdiphen; Phthalide; Picoxystrobin; Piperalin; Polyoxins; Polyoxorim; Probenazole; Prochloraz; Procymidone; Propamocarb; Propanosine-sodium; Propiconazole; Propineb; Proquinazid; Prothioconazole; Pyraclostrobin; Pyrazophos; Pyrifenox; Pyrimethanil; Pyroquilon; Pyroxyfur; Pyrrolnitrine; Quinconazole; Quinoxyfen; Quintozene; Simecoriazole; Spiroxamine; Sulfur; Tebuconazole; Tecloftalam; Tecnazene; Tetcyclacis; Tetraconazole; Thiabendazole; Thicyofen; Thifluzamide; Thiophanate-methyl; Thiram; Tioxymid; Tolclofos-methyl; Tolylfluanid; Triadimefon; Triadimenol; Triazbutil; Triazoxide; Tricyclamide; Tricyclazole; Tridemorph; Trifloxystrobin; Triflumizole; Triforine; Triticonazole; Uniconazole; Validamycin A; Vinclozolin; Zineb; Ziram; Zoxamide; (2S)-N-[2-[4-[[3-(4-Chlorphenyl)-2-propnyl]oxy]-3-methoxyphenyl]ethyl]-3-methyl-2-[(methylsulfonyl)amino]-butanamid; 1-(1-Naphthalinyl)-1H-pyrrol-2,5-dion; 2,3,5,6-Tetrachlor-4-(methylsulfonyl)-pyridin; 2-Amino-4-methyl-N-phenyl-5-thiazolcarboxamid; 2-Chlor-N-(2,3-dihydro-1,1,3-trimethyl-1H-inden-yl)-3-pyridincarboxamid; 3,4,5-Trichlor-2,6-pyridindicarbonitril; Actinovate; cis-1-(4-Chlorphenyl)-2-(1H-1,2,4-triazol-1-yl)cycloheptanol; Methyl 1-(2,3-dihydro-2,2-dimethyl-1H-inden-1-yl)-1H-imidazol-5-carboxylat; Monokaliumcarbonat; N-(6-Methoxy-3-pyridinyl)-cyclopropancarboxamid; N-Butyl-8-(1,1-dimethylethyl)-1-oxa-spiro[4.5]decan-3-amin; Natriumtetracarbonat;
sowie Kupfersalze und -zubereitungen, wie Bordeaux Mischung; Kupferhydroxid, Kupfernaphthenat; Kupferoxychlorid; Kupfersulfat; Cufraneb; Kupferoxid; Mancopper; Kupferoxin.

### Bakterizide:

Bronopol, Dichlorophen, Nitrapyrin, Nickel-Dimethyldithiocarbamat, Kasugamycin, Octhilinon, Furancarbonsäure, Oxytetracyclin, Probenazol, Streptomycin, Tecloftalam, Kupfersulfat und andere Kupfer-Zubereitungen.

### Insektizide / Akarizide / Nematizide:

*1. Acetylcholinesterase (AChE) Inhibitoren*
   1.1 Carbamate (z.B. Alanycarb, Aldicarb, Aldoxycarb, Allyxycarb, Aminocarb, Azamethiphos, Bendiocarb, Benfuracarb, Bufencarb, Butacarb, Butocarboxim, Butoxycarboxim, Carbaryl, Carbofuran, Carbosulfan, Chloethocarb, Coumaphos, Cyanofenphos, Cyanophos, Dimetilan, Ethiofencarb, Fenobucarb, Fenothiocarb, Formetanate, Furathiocarb, Isoprocarb, Metam-sodium, Methiocarb, Methomyl, Metolcarb, Oxamyl, Pirimicarb, Promecarb, Propoxur, Thiodicarb, Thiofanox, Triazamate, Trimethacarb, XMC, Xylylcarb)
   1.2 Organophosphate (z.B. Acephate, Azamethiphos, Azinphos (-methyl, -ethyl), Bromophosethyl, Bromfenvinfos (-methyl), Butathiofos, Cadusafos, Carbophenothion, Chlorethoxyfos, Chlorfenvinphos, Chlormephos, Chlorpyrifos (-methyl/-ethyl), Coumaphos, Cyanofenphos, Cyanophos, Chlorfenvinphos, Demeton-S-methyl, Demeton-S-methylsulphon, Dialifos, Diazinon, Dichlofenthion, Dichlorvos/DDVP, Dicrotophos, Dimethoate, Dimethylvinphos, Dioxabenzofos, Disulfoton, EPN, Ethion, Ethoprophos, Etrimfos, Famphur, Fenamiphos, Fenitrothion, Fensulfothion, Fenthion, Flupyrazofos, Fonofos, Formothion, Fosmethilan, Fosthiazate, Heptenophos, Iodofenphos, Iprobenfos, Isazofos, Isofenphos, Isopropyl O-salicylate, Isoxathion, Malathion, Mecarbam, Methacrifos, Methamidophos, Methidathion, Mevinphos, Monocrotophos, Naled, Omethoate, Oxydemeton-methyl, Parathion (-methyl/-ethyl), Phenthoate, Phorate, Phosalone, Phosmet, Phosphamidon, Phosphocarb, Phoxim, Pirimiphos (-methyl/-ethyl), Profenofos, Propaphos, Propetamphos, Prothiofos, Prothoate, Pyraclofos, Pyridaphenthion, Pyridathion, Quinalphos, Sebufos, Sulfotep, Sulprofos, Tebupirimfos, Temephos, Terbufos, Tetrachlorvinphos, Thiometon, Triazophos, Triclorfon, Vamidothion)
*2. Natrium-Kanal-Modulatoren* /*Spannungsabhängige Natrium-Kanal-Blocker*
   2.1 Pyrethroide (z.B. Acrinathrin, Allethrin (d-cis-trans, d-trans), Beta-Cyfluthrin, Bifenthrin, Bioallethrin, Bioallethrin-S-cyclopentyl-isomer, Bioethanomethrin, Biopermethrin, Bioresmethrin, Chlovaporthrin, Cis-Cypermethrin, Cis-Resmethrin, Cis-Permethrin, Clocythrin, Cycloprothrin, Cyfluthrin, Cyhalothrin, Cypermethrin (alpha-, beta-, theta-, zeta-), Cyphenothrin, DDT, Deltamethrin, Empenthrin (IR-isomer), Esfenvalerate, Etofenprox, Fenfluthrin, Fenpropathrin, Fenpyrithrin, Fenvalerate, Flubrocythrinate, Flucythrinate, Flufenprox, Flumethrin, Fluvalinate, Fubfenprox, Gamma-Cyhalothrin, Imiprothrin, Kadethrin, Lambda-Cyhalothrin, Metofluthrin, Permethrin (cis-, trans-), Phenothrin (1R-trans isomer), Prallethrin, Profluthrin, Protrifenbute, Pyresmethrin, Resmethrin, RU 15525, Silafluofen, Tau-Fluvalinate, Tefluthrin, Terallethrin, Tetramethrin (IR-isomer), Tralomethrin, Transfluthrin, ZXI 8901, Pyrethrins (pyrethrum))
   2.2 Oxadiazine (z.B. Indoxacarb)
*3. Acetylcholin-Rezeptor-Agonistenl- Antagonisten*
   3.1 Chloronicotinyle/Neonicotinoide (z.B. Acetamiprid, Clothianidin, Dinotefuran, Imidacloprid, Nitenpyram, Nithiazine, Thiacloprid, Thiamethoxam)
   3.2 Nicotine, Bensultap, Cartap
*4. Acetylcholin-Rezeptor-Modulatoren*
   4.1 Spinosyne (z.B. Spinosad)
*5. GABA-gesteuerte Chlorid-Kanal-Antagonisten*
   5.1 Cyclodiene Organochlorine (z.B. Camphechlor, Chlordane, Endosulfan, Gamma-HCH, HCH, Heptachlor, Lindane, Methoxychlor
   5.2 Fiprole (z.B. Acetoprole, Ethiprole, Fipronil, Vaniliprole)
*6. Chlorid-Kanal-Aktivatoren*
   6.1 Mectine (z.B. Abamectin, Avertnectin, Emamectin, Emamectin-benzoate, Ivermectin, Milbemectin, Milbemycin)
*7. Juvenilhornion-Mimetika* (z.B. Diofenolan, Epofenonane, Fenoxycarb, Hydroprene, Kinoprene, Methoprene, Pyriproxifen, Triprene)
*8. Ecdysonagonisten*/*disruptoren*
   8.1 Diacylhydrazine (z.B. Chromafenozide, Halofenozide, Methoxyfenozide, Tebufenozide)
*9. Inhibitoren der Chitinbiosynthese*
   9.1 Benzoylharnstoffe (z.B. Bistrifluron, Chlofluazuron, Diflubenzuron, Fluazuron, Flucycloxuron, Flufenoxuron, Hexaflumuron, Lufenuron, Novaluron, Noviflumuron, Penfluron, Teflubenzuron, Triflumuron)
   9.2 Buprofezin
   9.3 Cyromazine
*10. Inhibitoren der oxidativen Phosphorylierung, ATP-Disruptoren*
   10.1 Diafenthiuron
   10.2 Organotine (z.B. Azocyclotin, Cyhexatin, Fenbutatin-oxide)
*11*. *Entkoppler der oxidativen Phoshorylierung durch Unterbrechung des H-Protongradienten*
   11.1 Pyrrole (z.B. Chlorfenapyr)
   11.2 Dinitrophenole (z.B. Binapacyrl, Dinobuton, Dinocap, DNOC)
*12. Site-I-Elektronentransportinhibitoren*
   12.1 METI's (z.B. Fenazaquin, Fenpyroximate, Pyrimidifen, Pyridaben, Tebufenpyrad, Tolfenpyrad)
   12.2 Hydramethylnone
   12.3 Dicofol
*13. Site-II-Elektronentransportinhibitoren*
   13.1 Rotenone
*14. Site-III-Elektronentransportinhibitoren*
   14.1 Acequinocyl, Fluacrypyrim
*15. Mikrobielle Disruptoren der Insektendarmmembran*
   Bacillus thuringiensis-Stämme
*16. Inhibitoren der Fettsynthese*
   16.1 Tetronsäuren (z.B. Spirodiclofcn, Spiromesifen)
   16.2 Tetramsäuren [z.B. 3-(2,5-Dimethylphenyl)-8-methoxy-2-oxo-1-azaspiro[4.5]dec-3-en-4-yl ethyl carbonate (alias: Carbonic acid, 3-(2,5-dimethylpheny))-8-methoxy-2-oxo-1-azaspiro[4.5]dec-3-en-4-yl ethyl ester, CAS-Reg.-No.: 382608-10-8) and Carbonic acid, cis-3-(2,5-dimethylphenyl)-8-methoxy-2-oxo-1-azaspiro[4.5]dec-3-en-4-yl ethyl ester (CAS-Reg.-No.: 203313-25-1)]
*17. Carboxamide*
   (z.B. Flonicamid)
*18. Oktopaminerge Agonisten*
   (z.B. Amitraz)
*19. Inhibitoren der Magnesium-stimulierten ATPase*
   (z.B. Propargite)
*20. Phthalamide*
   (z.B. N²-[1,1-Dimethyl-2-(methylsulfonyl)ethyl]-3-iod-N¹-[2-methyl-4-[1,2,2,2-tetrafluor-1-(trifluormethyl)ethyl]phenyl]-1,2-benzenedicarboxamide (CAS-Reg.-No.: 272451-65-7), Flubendiamide)
*21. Nereistoxin-Analoge*
   (z.B. Thiocyclam hydrogen oxalate, Thiosultap-sodium)
*22. Biologika, Hormone oder Pheromone*
   (z.B. Azadirachtin, Bacillus spec., Bcauveria spec., Codlemone, Metarrhizium spec., Paecilomyces spec., Thuringiensin, Verticillium spec.)
*23. Wirkstoffe mit unbekannten oder nicht spezifischen Wirkmechanismen*
   23.1 Begasungsmittel (z.B. Aluminium phosphide, Methyl bromide, Sulfuryl fluoride)
   23.2 Selektive Fraßhemmer (z.B. Cryolite, Flonicamid, Pymetrozine)
   23.3 Milbenwachstumsinhibitoren (z.B. Clofentezine, Etoxazole, Hexythiazox)
   23.4 Amidoflumet, Benclothiaz, Benzoximate, Bifenazate, Bromopropylate, Buprofezin, Chinomethionat, Chlordimeform, Chlorobenzilate, Chloropicrin, Clothiazoben, Cycloprene, Cyflumetofen, Dicyclanil, Fenoxacrim, Fentrifanil, Flubenzimine, Flufenerim, Flutenzin, Gossyplure, Hydramethylnone, Japonilure, Metoxadiazone, Petroleum, Piperonyl butoxide, Potassium oleate, Pyrafluprole, Pyridalyl, Pyriprole, Sulfluramid, Tetradifon, Tetrasul, Triarathene, Verbutin,
ferner die Verbindung 3-Methyl-phenyl-propylcarbamat (Tsumacide Z), die Verbindung 3-(5-Chlor-3-pyridinyl)-8-(2,2,2-trifluorethyl)-8-azabicyclo[3.2.1]octan-3-carbonitril (CAS-Reg.-Nr. 185982-80-3) und das entsprechende 3-endo-Isomere (CAS-Reg.-Nr. 185984-60-5) (vgl. WO 96/37494, WO 98/25923), sowie Präparate, welche insektizid wirksame Pflanzenextrakte, Nematoden, Pilze oder Viren enthalten.

Auch eine Mischung mit anderen bekannten Wirkstoffen, wie Herbiziden oder mit Düngemitteln und Wachstumsregulatoren, Safener bzw. Semiochemicals ist möglich.

Darüber hinaus weisen die erfindungsgemäßen Verbindungen der Formel (I) auch sehr gute antimykotische Wirkungen auf. Sie besitzen ein sehr breites antimykotisches Wirkungsspektrum, insbesondere gegen Dermatophyten und Sprosspilze, Schimmel und diphasische Pilze (z.B. gegen Candida-Spezies wie Candida albicans, Candida glabrata) sowie Epidermophyton floccosum, Aspergillus-Spezies wie Aspergillus niger und Aspergillus fumigatus, Trichophyton-Spezies wie Trichophyton mentagrophytes, Microsporon-Spezies wie Microsporon canis und audouinii. Die Aufzählung dieser Pilze stellt keinesfalls eine Beschränkung des erfassbaren mykotischen Spektrums dar, sondern hat nur erläuternden Charakter.

Die Wirkstoffe können als solche, in Form ihrer Formulierungen oder den daraus bereiteten Anwendungsformen, wie gebrauchsfertige Lösungen, Suspensionen, Spritzpulver, Pasten, lösliche Pulver, Stäubemittel und Granulate angewendet werden. Die Anwendung geschieht in üblicher Weise, z.B. durch Gießen, Verspritzen, Versprühen, Verstreuen, Verstäuben, Verschäumen, Bestreichen usw. Es ist ferner möglich, die Wirkstoffe nach dem Ultra-Low-Volume-Verfahren auszubringen oder die Wirkstoffzubereitung oder den Wirkstoff selbst in den Boden zu injizieren. Es kann auch das Saatgut der Pflanzen behandelt werden.

Beim Einsatz der erfindungsgemäßen Wirkstoffe als Fungizide können die Aufwandmengen je nach Applikationsart innerhalb eines größeren Bereiches variiert werden. Bei der Behandlung von Pflanzenteilen liegen die Aufwandmengen an Wirkstoff im allgemeinen zwischen 0,1 und 10.000 g/ha, vorzugsweise zwischen 10 und 1.000 g/ha. Bei der Saatgutbehandlung liegen die Aufwandmengen an Wirkstoff im allgemeinen zwischen 0,001 und 50 g pro Kilogramm Saatgut, vorzugsweise zwischen 0,01 und 10 g pro Kilogramm Saatgut. Bei der Behandlung des Bodens liegen die Aufwandmengen an Wirkstoff im allgemeinen zwischen 0, I und 10.000 g/ha, vorzugsweise zwischen I und 5.000 g/ha.

Wie bereits oben erwähnt, können erfindungsgemäß alle Pflanzen und deren Teile behandelt werden. In einer bevorzugten Ausführungsform werden wild vorkommende oder durch konventionelle biologische Zuchtmethoden, wie Kreuzung oder Protoplastenfusion erhaltenen Pflanzenarten und Pflanzensorten sowie deren Teile behandelt. In einer weiteren bevorzugten Ausführungsform werden transgene Pflanzen und Pflanzensorten, die durch gentechnologische Methoden gegebenenfalls in Kombination mit konventionellen Methoden erhalten wurden (Genetically Modified Organisms) und deren Teile behandelt. Der Begriff "Teile" bzw. "Teile von Pflanzen" oder "Pflanzenteile" wurde oben erläutert.

Besonders bevorzugt werden erfindungsgemäß Pflanzen der jeweils handelsüblichen oder in Gebrauch befindlichen Pflanzensorten behandelt. Unter Pflanzensorten versteht man Pflanzen mit neuen Eigenschaften ("Traits"), die sowohl durch konventionelle Züchtung, durch Mutagenese oder durch rekombinante DNA-Techniken gezüchtet worden sind. Dies können Sorten, Rassen, Bio- und Genotypen sein.

Je nach Pflanzenarten bzw. Pflanzensorten, deren Standort und Wachstumsbedingungen (Böden, Klima, Vegetationsperiode, Ernährung) können durch die erfindungsgemäße Behandlung auch überadditive ("synergistische") Effekte auftreten. So sind beispielsweise erniedrigte Aufwandmengen und/oder Erweiterungen des Wirkungsspektrums und/oder eine Verstärkung der Wirkung der erfindungsgemäß verwendbaren Stoffe und Mittel, besseres Pflanzenwachstum, erhöhte Toleranz gegenüber hohen oder niedrigen Temperaturen, erhöhte Toleranz gegen Trockenheit oder gegen Wasser- bzw. Bodensalzgehalt, erhöhte Blühleistung, erleichterte Ernte, Beschleunigung der Reife, höhere Ernteerträge, höhere Qualität und/oder höherer Emährungswert der Ernteprodukte, höhere Lagerfähigkeit und/oder Bearbeitbarkeit der Ernteprodukte möglich, die über die eigentlich zu erwartenden Effekte hinausgehen.

Zu den bevorzugten erfindungsgemäß zu behandelnden transgenen (gentechnologisch erhaltenen) Pflanzen bzw. Pflanzensorten gehören alle Pflanzen, die durch die gentechnologische Modifikation genetisches Material erhielten, welches diesen Pflanzen besondere vorteilhafte wertvolle Eigenschaften ("Traits") verleiht. Beispiele für solche Eigenschaften sind besseres Pflanzenwachstum, erhöhte Toleranz gegenüber hohen oder niedrigen Temperaturen, erhöhte Toleranz gegen Trockenheit oder gegen Wasser- bzw. Bodensalzgehalt, erhöhte Blühleistung, erleichterte Ernte, Beschleunigung der Reife, höhere Ernteerträge, höhere Qualität und/oder höherer Ernährungswert der Ernteprodukte, höhere Lagerfähigkeit und/oder Bearbeitbarkeit der Ernteprodukte. Weitere und besonders hervorgehobene Beispiele für solche Eigenschaften sind eine erhöhte Abwehr der Pflanzen gegen tierische und mikrobielle Schädlinge, wie gegenüber Insekten, Milben, pflanzenpathogenen Pilzen, Bakterien und/oder Viren sowie eine erhöhte Toleranz der Pflanzen gegen bestimmte herbizide Wirkstoffe. Als Beispiele transgener Pflanzen werden die wichtigen Kulturpflanzen, wie Getreide (Weizen, Reis), Mais, Soja, Kartoffel, Baumwolle, Tabak, Raps sowie Obstpflanzen (mit den Früchten Äpfel, Birnen, Zitrusfrüchten und Weintrauben) erwähnt, wobei Mais, Soja, Kartoffel, Baumwolle, Tabak und Raps besonders hervorgehoben werden. Als Eigenschaften ("Traits") werden besonders hervorgehoben die erhöhte Abwehr der Pflanzen gegen Insekten, Spinnentiere, Namatoden und Schnecken durch in den Pflanzen entstehende Toxine, insbesondere solche, die durch das genetische Material aus Bacillus Thuringiensis (z.B. durch die Gene CryIA(a), CryIA(b), CryIA(c), CryllA, CryIIIA, CryIIIB2, Cry9c Cry2Ab, Cry3Bb und CryIF sowie deren Kombinationen) in den Pflanzen erzeugt werden (im folgenden "Bt Pflanzen"). Als Eigenschaften ("Traits") werden auch besonders hervorgehoben die erhöhte Abwehr von Pflanzen gegen Pilze, Bakterien und Viren durch Systemische Akquirierte Resistenz (SAR), Systemin, Phytoalexine, Elicitoren sowie Resistenzgene und entsprechend exprimierte Proteine und Toxine. Als Eigenschaften ("Traits") werden weiterhin besonders hervorgehoben die erhöhte Toleranz der Pflanzen gegenüber bestimmten herbiziden Wirkstoffen, beispielsweise Imidazolinonen, Sulfonylharnstoffen, Glyphosate oder Phosphinotricin (z.B. "PAT"-Gen). Die jeweils die gewünschten Eigenschaften ("Traits") verleihenden Gene können auch in Kombinationen miteinander in den transgenen Pflanzen vorkommen. Als Beispiele für "Bt Pflanzen" seien Maissorten, Baumwollsorten, Sojasorten und Kartoffelsorten genannt, die unter den Handelsbezeichnungen YIELD GARD® (z.B. Mais, Baumwolle, Soja), KnockOut® (z.B. Mais), StarLink® (z.B. Mais), Bollgard® (Baumwolle), Nucoton® (Baumwolle) und NewLeaf® (Kartoffel) vertrieben werden. Als Beispiele für Herbizid tolerante Pflanzen seien Maissorten, Baumwollsorten und Sojasorten genannt, die unter den Handelsbezeichnungen Roundup Ready® (Toleranz gegen Glyphosate z.B. Mais, Baumwolle, Soja), Liberty Link® (Toleranz gegen Phosphinotricin, z.B. Raps), IMI® (Toleranz gegen Imidazolinone) und STS® (Toleranz gegen Sulfonylharnstoffe z.B. Mais) vertrieben werden. Als Herbizid resistente (konventionell auf Herbizid-Toleranz gezüchtete) Pflanzen seien auch die unter der Bezeichnung Clearfield® vertriebenen Sorten (z.B. Mais) erwähnt. Selbstverständlich gelten diese Aussagen auch für in der Zukunft entwickelte bzw. zukünftig auf den Markt kommende Pflanzensorten mit diesen oder zukünftig entwickelten genetischen Eigenschaften ("Traits").

Die aufgeführten Pflanzen können besonders vorteilhaft erfindungsgemäß mit den Verbindungen der allgemeinen Formel (I) bzw. den erfindungsgemäßen Wirkstoffmischungen behandelt werden. Die bei den Wirkstoffen bzw. Mischungen oben angegebenen Vorzugsbereiche gelten auch für die Behandlung dieser Pflanzen. Besonders hervorgehoben sei die Pflanzenbehandlung mit den im vorliegenden Text speziell aufgeführten Verbindungen bzw. Mischungen.

Weiterhin eignen sich die erfindungsgemäßen Verbindungen der Formel (I) zur Unterdrückung des Wachstums von Tumorzellen in Menschen und Säugetieren. Dies basiert auf einer Wechselwirkung der erfindungsgemäßen Verbindungen mit Tubulin und Mikrotubuli und durch Förderung der Mikrotubuli-Polymerisation.

Zu diesem Zweck kann man eine wirksame Menge an einer oder mehreren Verbindungen der Formel (I) oder pharmazeutisch verträglicher Salze davon verabreichen.

Die Herstellung und die Verwendung der erfindungsgemäßen Wirkstoffe geht aus den folgenden Beispielen hervor.

### Herstellungsbeispiele

### Beispiel 1

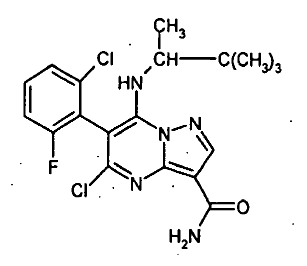

Verfahren (a, Variante α)

5 ml verdünnte Schwefelsäure und 0,4 g (0,985 mmol) 3-Cyano-5-chlor-6-(2-chlor-6-fluorphenyl)-7-(3,3-dimethyl-but-2-yl-amino)-pyrazolo-[1,5-a]pyrimidin werden bei 0°C verrührt und danach weitere 5 Stunden bei Raumtemperatur gerührt. Anschließend wird das Reaktionsgemisch auf Eis gegossen. Der anfallende Niederschlag wird abgesaugt, mit Wasser gewaschen und getrocknet. Man erhält auf diese Weise 0,32 g (61,28 % der Theorie) an 3-Amido-5-chlor-6-(2-chlor-6-fluor-phenyl)-7-(3,3-dimethyl-but-2-yl-amino)-pyrazolo-[1,5-a]pyrimdin.
HPLC: logP = 3,62

### Beispiel 2

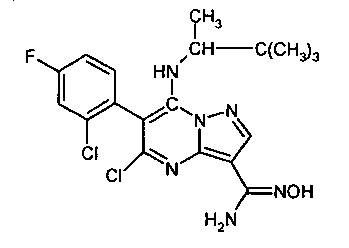

Verfahren (a, Variante β)

Ein Gemisch aus 0,400 g (0,985 mmol) 3-Cyano-5-chlor-6-(2-chlor-4-fluorphenyl)-7-(3,3-dimethyl-but-2-yl-amino)-pyrazolo[1,5-a]pyrimidin und 20 ml Ethanol wird bei Raumtemperatur mit 0,082g (1,181 mmol) Hydroxylammoniumchlorid und 0,800g Amberlyst A-21 versetzt und 16 Stunden bei Raumtemperatur geschüttelt. Danach wird das Reaktionsgemisch erneut mit 40 mg Hydroxylammoniumchlorid und 200 mg Amberlyst A-21 versetzt und 48 Stunden beim Raumtemperatur geschüttelt. Es wird aufgearbeitet, indem man das enthaltene Festprodukt absaugt, die Mutterlauge unter vermindertem Druck einengt und den verbleibenden Rückstand mit Petrol-eiher:Methyl.-tert.-butyl-ether = 4:1 an Kieselgel chromatographiert. Man erhält auf diese Weise 0,16 g (36,47 % der Theorie) an 3-Hydroximinoamido-5-chlor-6-(2-chlor-4-fluor-phenyl)-7-(3,3-dimethyl-but-2-yl-amino)-pyrazolo[1,5-a]pyrimidin.
HPLC: logP = 2,55

### Beispiel 3

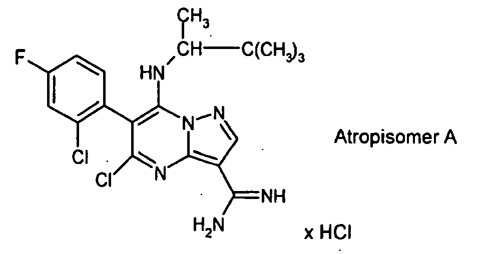

Verfahren (a, Variante γ)

Ein Gemisch aus 1,000 g (2,461 mmol) 3-Cyano-5-chlor-(2-chlor-4-fluor-phenyl)-7-(3,3-dimethyl-but-2-yl-amino)-pyrazolo[1,5-a]pyrimidin und 5 ml Methanol wird bei Raumtemperatur mit 0,053 g (0,246 mmol) Natrium-methylat in Methanol versetzt und 48 Stunden bei Raumtemperatur gerührt. Danach fügt man 0,132 g (2,461 mmol) Ammoniumchlorid hinzu und rührt 24 Stunden bei Raumtemperatur. Anschließend wird das Reaktionsgemisch unter vermindertem Druck eingeengt. Man erhält auf diese Weise 0,95 g eines Produktes, das zu 8,6 % aus 3-Iminoamid-5-chlor-6-(2-chlor-4-fluor-phenyl)-7-(3,3-dimethyl-but-2-yl-amino)-pyrazolo[1,5-a]pyrimidin-Hydrochlorid in Form des Atropisomeren A besteht.
HPLC: logP = 2,72

### Beispiel 4

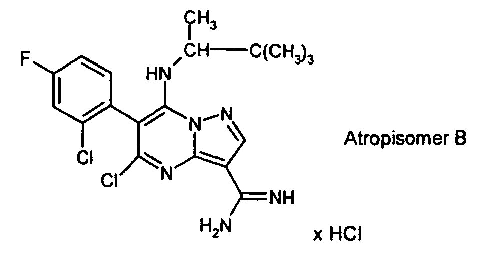

Verfahren (a, Variante γ)

Ein Gemisch aus 1,0 g (2,461 mmol) 3-Cyano-5-chlor-6-(2-chlor-4-fluor-phenyl)-7-(3,3-dimethy)-but-2-yl-amino)-pyrazolo[1,5-a]-pyrimidin und 25 ml Methanol wird bei Raumtemperatur mit 0,053 g (0,246 mmol) Natrium-methylat in Methanol versetzt und 48 Stunden bei Raumtemperatur gerührt. Danach fügt man 0,132 g (2,461 mmol) Ammoniumchlorid hinzu und rührt 24 Stunden bei Raumtemperatur. Anschließend wird das Reaktionsgemisch unter vermindertem Druck eingeengt. Man erhält auf diese Weise 0,98 g eines Produktes, das zu 15,24 % aus 3-Iminoamid-5-Chlor-6-(2-chlor-4-chlor-phenyl)-7-(3,3-dimethyl-but-2-yl-amino)-pyrazolo[1,5-a]-pyrirnidin-Hydrochlorid in Form des Atropisomeren B besteht.
HPLC: logP = 2,58

### Beispiel 5

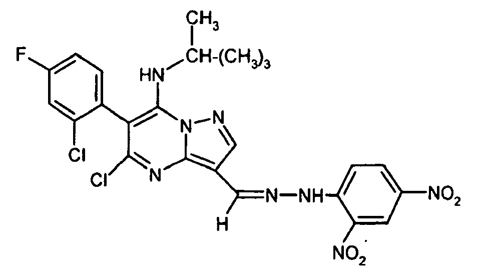

Verfahren (b)

48 mg (0,244 mmol) 2,4-Dinitrophenylhydrazin werden bei Raumtemperatur unter Rühren mit 0,2 ml konzentrierter Schwefelsäure und tropfenweise mit 0,3 ml Wasser versetzt. Anschließend werden unter Rühren zunächst 1 ml Ethanol und dann eine 10 %ige Lösung von 0, 1 g (0,244 mmol) 3-Formyl-5-chlor-6-(2-chlor-4-fluor-phenyl)-7-(3,3-dimethy)-but-2-yl-amino)-pyrazolo[1,5-a]pyrimidin hinzugefügt. Das entstandene Festprodukt wird abgesaugt, mit Wasser gewaschen und getrocknet. Man erhält auf diese Weise 0,09 g (56,25 % der Theorie) an 3-(2,4-Dinitriophenyl-hydrazimino-methyl)-5-chlor-6(2-chlor-4-fluor-phenyl)-7-(3,3-dimethyl-but-2-yl-amino)-pyrazolo[1,5-a]pyrimidin.
HPLC: logP = 6,28

Nach den zuvor angegebenen Methoden werden auch die in der folgenden Tabelle I aufgeführten Stoffe der Formel 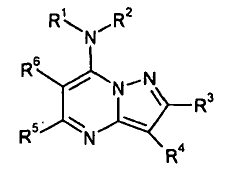 hergestellt.

**Tabelle 1**

| **Bsp.-Nr.** | 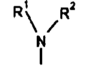 | **R³** | **R⁴** | **R⁵** | **R⁶** | **logP** |
|---|---|---|---|---|---|---|
| 6 | 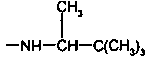 | H | 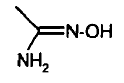 | Cl | 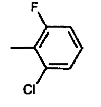 | 2,26 |
| 7 | 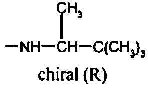 | H | 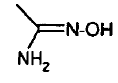 | Cl | 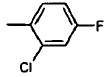 | 2,47 |
| 8 | 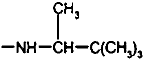 | H | 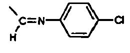 | Cl | 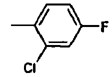 | MH⁺=518 |
| 9 | 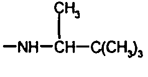 | H | 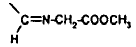 | Cl | 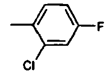 | MH⁺=480 |
| 10 | -NH₂ | H | 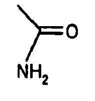 | Cl | 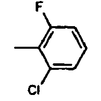 | 1,82 |
| 11 | -NH₂ | H | 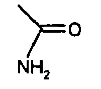 | Cl | 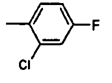 | 2,03 |
| 12 | 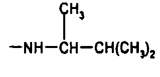 | H | 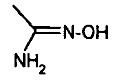 | Cl | 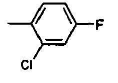 | 2,34 |
| 13 | 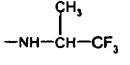 | H | 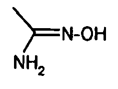 | Cl | 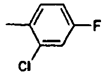 | 2,1 |
| 14 | 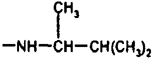 | H | 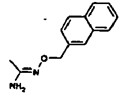 | Cl | 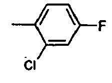 | 5,14 |
| 15 | 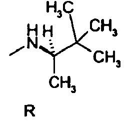 | H | 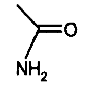 | Cl | 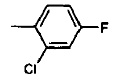 | 3,88 |
| 16 | 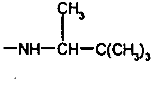 | H | 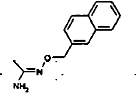 | Cl | 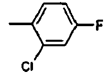 | 5,69 |
| 17 | 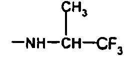 | H | 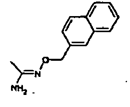 | Cl | 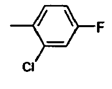 | 4,99 |
| 18 | 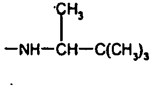 | H | 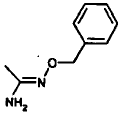 | Cl | 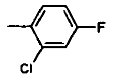 | 4,97 |
| 19 | 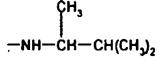 | H | 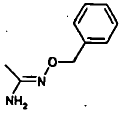 | Cl | 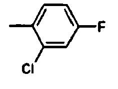 | 4,49 |
| 20 | 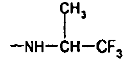 | H | 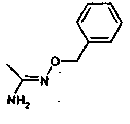 | Cl | 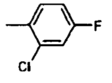 | 4,27 |
| 21 | 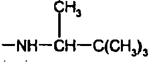 | H | 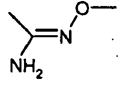 | Cl | 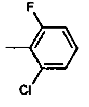 | |
| 22 | 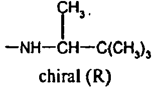 | H | 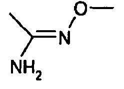 | Cl | 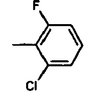 | |
| 23 | 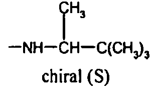 | H | 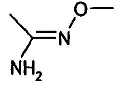 | Cl | 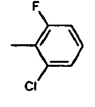 | |
| 24 | 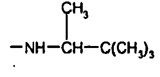 | H | 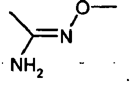 | Cl | 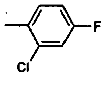 | |
| 25 | 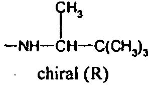 | H | 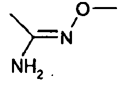 | Cl | 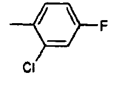 | |
| 26 | 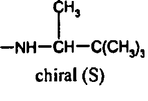 | H | 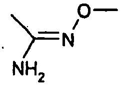 | Cl | 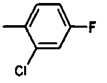 | |
| 27 | 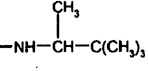 | H | 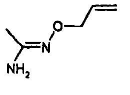 | Cl | 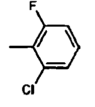 | |
| 28 | 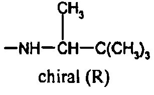 | H | 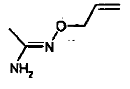 | Cl | 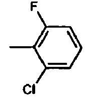 | |
| 29 | 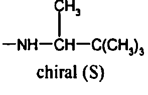 | H | 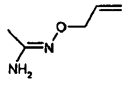 | Cl | 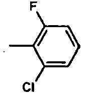 | |
| 30 | 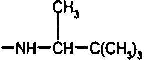 | H | 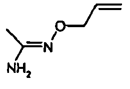 | Cl | 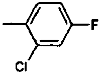 | |
| 31 | 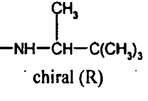 | H | 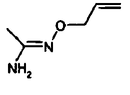 | Cl | 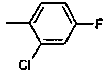 | |
| 32 | 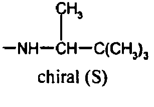 | H | 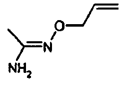 | Cl | 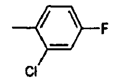 | |
| 33 | 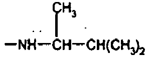 | H | 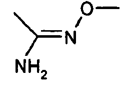 | Cl | 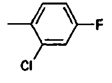 | |
| 34 | 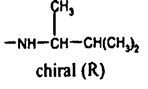 | H | 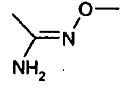 | Cl | 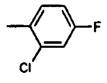 | |
| 35 | 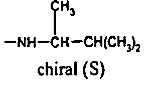 | H | 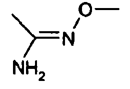 | Cl | 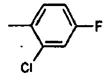 | |
| 42 | 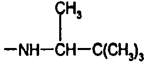 | H | 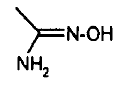 | Cl | 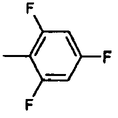 | |
| 43 | 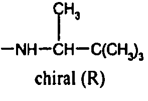 | H | 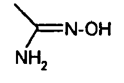 | Cl | 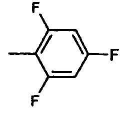 | |
| 44 | 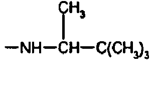 | H | 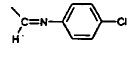 | Cl | 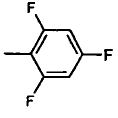 | |
| 45 | 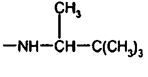 | H | 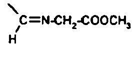 | Cl | 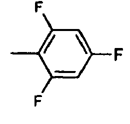 | |
| 46 | -NH₂ | H | 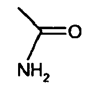 | Cl | 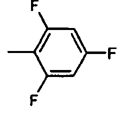 | |
| 47 | 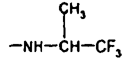 | H | 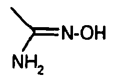 | Cl | 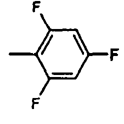 | |
| 48 | 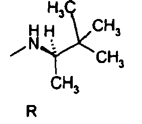 | H | 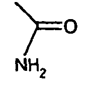 | Cl | 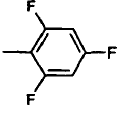 | |
| 49 | 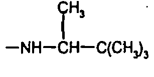 | H | 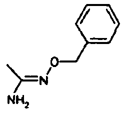 | Cl | 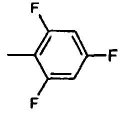 | |
| 50 | 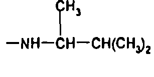 | H | 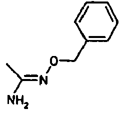 | Cl | 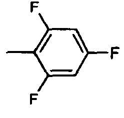 | |
| 51 | 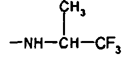 | H | 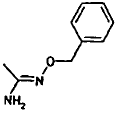 | Cl | 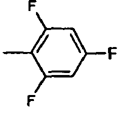 | |
| 52 | 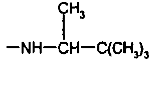 | H | 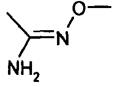 | Cl | 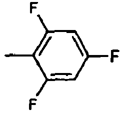 | |
| 53 | 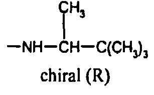 | H | 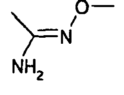 | Cl | 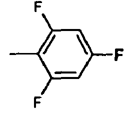 | |
| 54 | 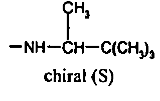 | H | 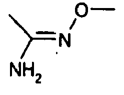 | Cl | 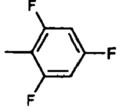 | |
| 55 | 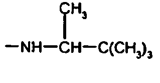 | H | 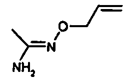 | Cl | 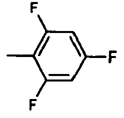 | |
| 56 | 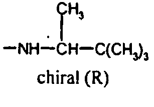 | H | 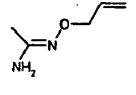 | Cl | 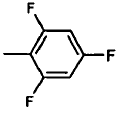 | |
| 57 | 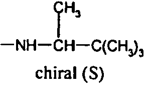 | H | 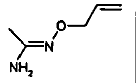 | Cl | 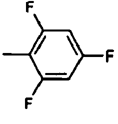 | |
| 58 | 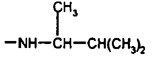 | H | 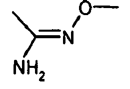 | Cl | 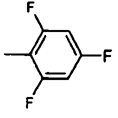 | |
| 59 | 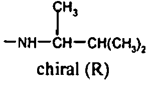 | H | 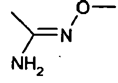 | Cl | 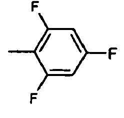 | |
| 60 | 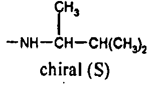 | H | 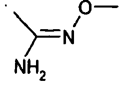 | Cl | 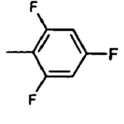 | |
| 61 | 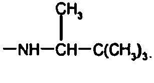 | H | 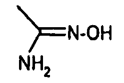 | Cl | 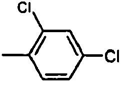 | |
| 62 | 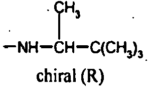 | H | 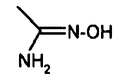 | Cl | 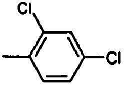 | |
| 63 | 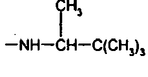 | H | 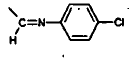 | Cl | 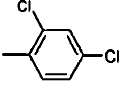 | |
| 64 | 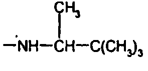 | H | 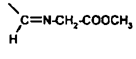 | Cl | 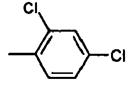 | |
| 65 | -NH₂ | H | 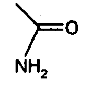 | Cl | 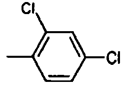 | |
| 66 | 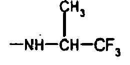 | H | 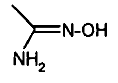 | Cl | 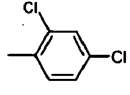 | |
| 67 | 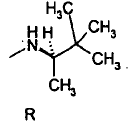 | H | 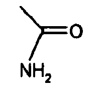 | Cl | 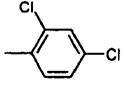 | |
| 68 | 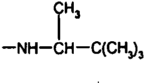 | H | 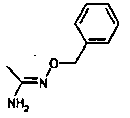 | Cl | 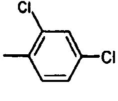 | |
| 69 | 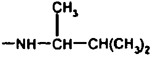 | H | 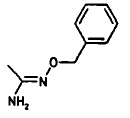 | Cl | 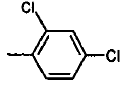 | |
| 70 | 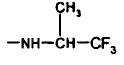 | H | 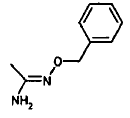 | Cl | 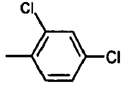 | |
| 71 | 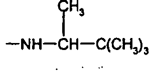 | H | 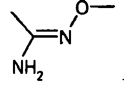 | Cl | 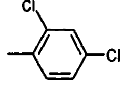 | |
| 72 | 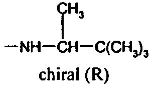 | H | 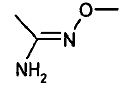 | Cl | 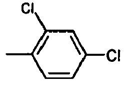 | |
| 73 | 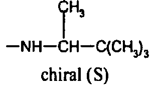 | H | 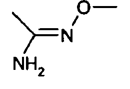 | Cl | 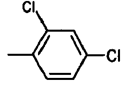 | |
| 74 | 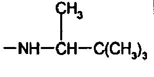 | H | 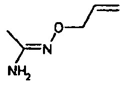 | Cl | 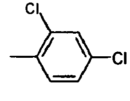 | |
| 75 | 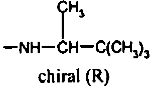 | H | 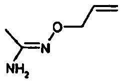 | Cl | 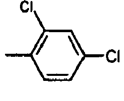 | |
| 76 | 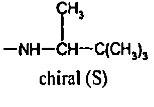 | H | 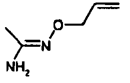 | Cl | 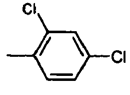 | |
| 77 | 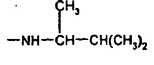 | H | 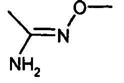 | Cl | 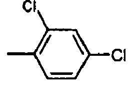 | |
| 78 | 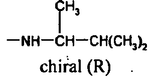 | H | 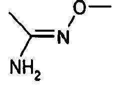 | Cl | 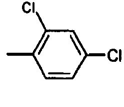 | |
| 79 | 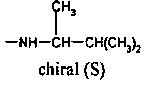 | H | 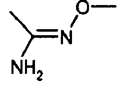 | Cl | 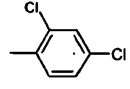 | |
| 80 | 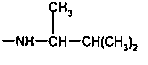 | H | 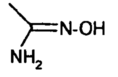 | Cl | 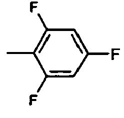 | |
| 81 | 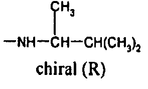 | H | 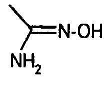 | Cl | 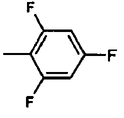 | |
| 82 | 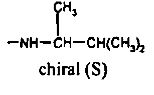 | H | 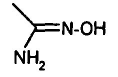 | Cl | 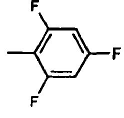 | |
| 83 | 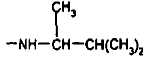 | H | 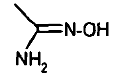 | Cl | 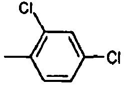 | |
| 84 | 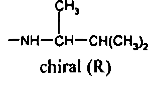 | H | 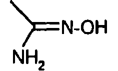 | CI | 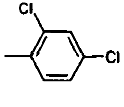 | |
| 85 | 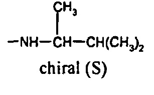 | H | 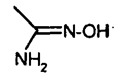 | Cl | 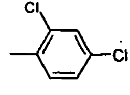 | |

Die Bestimmung der logP-Werte erfolgt gemäß EEC-Directive 79/831 Annex V. A8 durch HPLC (Gradientenmethode, Aceteonitril/0,1 % wässrige Phosphorsäure).

### Herstellung von Ausgangssubstanzen

### Beispiel 36

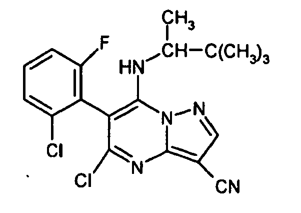

Ein Gemisch aus 10,0 g (29,27 mmol) 3-Cyano-5,7-dichlor-6-(2-chlor-6-fluor-phenyl)-pyrazolo[1,5-a]pyrimidin in 250 ml Acetonitril wird bei Raumtemperatur unter Rühren mit 7,407 g (73,194 mmol) 2-Amino-3,3-dimethyl-butan versetzt. Nach beendeter Zugabe wird das Reaktionsgemisch 3 Stunden bei Raumtemperatur gerührt und dann in ein Gemisch aus Wasser und Salzsäure eingerührt. Das dabei anfallende Festprodukt wird abgesaugt, mehrfach mit Wasser gewaschen und getrocknet. Man erhält auf diese Weise 10,8 g (90,79 % der Theorie) an 3-Cyano-5-chlor-6-(2-chlor-6-fluorphenyl)-7-(3,3-dimethyl-but-2-yl-amino)-pyrazolo[1,5-a]pyrimidin.
HPLC: logP = 4,59.

### Beispiel 37

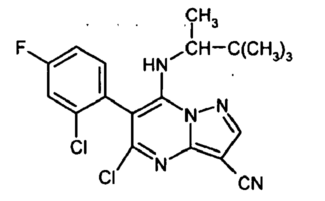

Die Herstellung der Verbindung der oben angegebenen Formel erfolgt nach der im Beispiel 18 angegebenen Methode.
HPLC: logP = 4,78

### Beispiel 38

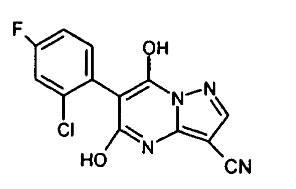

48 g (0,184 Mol) 2-Chlor-4-fluor-phenylmalonsäuredimethyleter werden 19,91 g (0,184 Mol) 4-Cyano-5-aminopyrazol und mit 37,55 g (0,203 Mol) Tri-n-butylamin vermischt und 6 Stunden bei 180°C gerührt. Das bei der Reaktion entstehende Methanol wird kontinuierlich abdestilliert. Anschließend wird das Reaktionsgemisch auf Raumtemperatur abgekühlt. Bei 95°C und 1 mbar werden flüchtige Komponenten abdestilliert. Man erhält als Rückstand 6-(2-chlor-4-fluorphenyl)-5,7-dihydroxypyrazolo[1,5-a]pyrimidin-3-carbonitril in Form eines Rohproduktes, das ohne zusätzliche Reinigung für die weitere Synthese verwendet wird.

### Beispiel 39

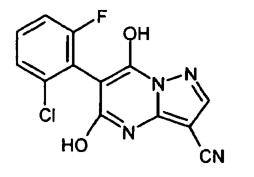

Die Herstellung der Verbindung der oben angegebenen Formel erfolgt nach der im Beispiel 22 beschriebenen Methode.
HPLC: logP = 0, 19

### Beispiel 40

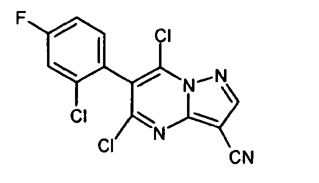

Das gemäß Beispiel 22 hergestellte 6-(2-Chlor-4-fluor-phenyl)-5,7-dihydroxy-pyrazolo[1,5-a]-pyrimidin-3-carbonitril wird im Rohzustand in 367,3 g (2,395 Mol) Phosphoroxychlorid gelöst.

Man gibt bei Raumtemperatur 31,95 g (0,153 Mol) Phosphorpentachlorid in Portionen dazu. Dann kocht man die Mischung 12 Stunden unter Rückfluss unter Rückfluss. Die flüchtigen Komponenten werden unter vermindertem Druck abdestilliert, der Rückstand wird mit Dichlormethan versetzt und mit Wasser gewaschen. Die organische Phase wird Ober Natriumsulfat getrocknet und unter vermindertem Druck eingeengt. Der Rückstand wird mit 3 Teilen Cyclohexan und 1 Teil Essigsäureethylester als Laufmittel an Kieselgel chromatographiert. Man erhält 21 g 95,7 %iges 3-Cyano-5,7-dichlor-6-(2-chlor-4-fluorphenyl)-pyrazolo[1,5-a]pyrimdin.
HPLC: logP = 3,49
¹H-NMR (DMSO-d6, Tetramethylsilan): δ = 7,44-7,52 (1H); 7,62-7,66 (1H); 7,71-7,77 (1H); 9,03 (1H) ppm.

### Beispiel 41

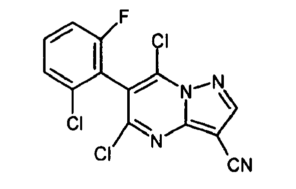

Die Herstellung der Verbindung der oben angegebenen Formel erfolgt nach der im Beispiel 24 beschriebenen Methode.
HPLC: logP = 3,31

### Verwendungsbeispiele

### Beispiel A

### Venturia - Test (Apfel) / protektiv

- Lösungsmittel:: 24,5 Gewichtsteile Aceton
24,5 Gewichtsteile Dimethylacetamid
- Emulgator:: 1,0 Gewichtsteile Alkyl-Aryl-Polyglykolether

Zur Herstellung einer zweckmäßigen Wirkstoffzubereitung vermischt man I Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit werden junge Pflanzen mit der Wirkstoffzubereitung in der angegebenen Aufwandmenge besprüht. Nach Antrocknen des Spritzbelages werden die Pflanzen mit einer wässrigen Konidiensuspension des Apfelschorferregers Venturia inaequalis inokuliert und verbleiben dann 1 Tag bei ca. 20°C und 100% relativer Luftfeuchtigkeit in einer Inkubationskabine.

Die Pflanzen werden dann im Gewächshaus bei ca. 21°C und einer relativen Luftfeuchtigkeit von ca. 90% aufgestellt.

10 Tage nach der Inokulation erfolgt die Auswertung. Dabei bedeutet 0% ein Wirkungsgrad, der demjenigen der Kontrolle entspricht, während ein Wirkungsgrad von 100% bedeutet, dass kein Befall beobachtet wird.

In diesem Test zeigten die in den Beispielen 1, 2, 7, 12 und 13 aufgeführten erfindungsgemäßen Verbindungen bei einer Aufwandmenge von 100 g/ha einen Wirkungsgrad von über 90 %.

### Beispiel B

### Botrytis - Test (Bohne) / protektiv

- Lösungsmittel:: 24,5 Gewichtsteile Aceton
24,5 Gewichtsteile Dimethylacetamid
- Emulgator:: 1,0 Gewichtsteile Alkyl-Aryl-Polyglykolether

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit werden junge Pflanzen mit der Wirkstoffzubereitung in der angegebenen Aufwandmenge besprüht. Nach Antrocknen des Spritzbelages werden auf jedes Blatt 2 kleine mit Botrytis cinerea bewachsene Agarstückchen aufgelegt. Die inokulierten Pflanzen werden in einer abgedunkelten Kammer bei ca. 20°C und 100% relativer Luftfeuchtigkeit aufgestellt.

2 Tage nach der Inokulation wird die Größe der Befallsflecken auf den Blättern ausgewertet. Dabei bedeutet 0% ein Wirkungsgrad, der demjenigen der Kontrolle entspricht, während ein Wirkungsgrad von 100% bedeutet, dass kein Befall beobachtet wird.

In diesem Test zeigten die in den Beispielen 1, 2, 7, 12 und 13 aufgeführten erfindungsgemäßen Verbindungen bei einer Aufwandmenge von 500 g/ha einen Wirkungsgrad von über 90 %.

### Beispiel C

### Sphaerotheca - Test (Gurke) / protektiv

- Lösungsmittel:: 49 Gewichtsteile N,N-Dimethylfromamid
- Emulgator:: 1 Gewichtsteil Alkylarylpolyglykolether

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit bespritzt man junge Gurkenpflanzen mit der Wirkstoffzubereitung in der angegebenen Aufwandmenge. 1 Tag nach der Behandlung werden die Pflanzen mit einer Sporensuspension von Sphaerotheca fuliginea inokuliert. Anschließend werden die Pflanzen in einem Gewächshaus bei 70% relativer Luftfeuchtigkeit und einer Temperatur von 23°C aufgestellt.

7 Tage nach der Inokulation erfolgt die Auswertung. Dabei bedeutet 0% ein Wirkungsgrad, der demjenigen der Kontrolle entspricht, während ein Wirkungsgrad von 100% bedeutet, dass kein Befall beobachtet wird.

In diesem Test zeigten die in den Beispielen 2 und 7 aufgeführten erfindungsgemäßen Verbindungen bei einer Aufwandmenge von 750 g/ha einen Wirkungsgrad von über 90 %.

## Patentansprüche

1. Pyrazolopyrimidine der Formel 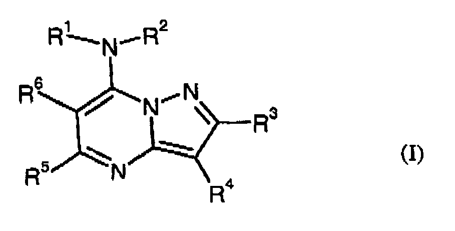 in denen
R¹ für Wasserstoff, Alkyl mit 1 bis 6 Kohlenstoffatomen steht, das einfach bis fünffach, gleichartig oder verschieden substituiert sein kann durch Halogen, Cyano, Hydroxy, Alkoxy mit 1 bis 4 Kohlenstoffatomen und/oder Cycloalkyl mit 3 bis 6 Kohlenstoffatomen,
R¹ für Alkenyl mit 2 bis 6 Koltlenstoffatomen steht, das einfach bis dreifach, gleichartig oder verschieden substituiert sein kann durch Halogen, Cyano, Hydroxy, Alkoxy mit 1 bis 4 Kohlenstoffatomen und/oder Cycloalkyl mit 3 bis 6 Kohlenstoffatomen, oder
R¹ für Alkinyl mit 3 bis 6 Kohlenstoffatomen steht, das einfach bis dreifach, gleichartig oder verschieden substituiert sein kann durch Halogen, Cyano, Alkoxy mit 1 bis 4 Kohlenstoffatomen und/oder Cycloalkyl mit 3 bis 6 Kohlenstoffatomen, oder
R¹ für Cycloalkyl mit 3 bis 6 Kohlenstoffatomen steht, das einfach bis dreifach, gleichartig oder verschieden substituiert sein kann durch Halogen und/oder Alkyl mit 1 bis 4 Kohlenstoffatomen, oder
R¹ für gesättigtes oder ungesättigtes Heterocyclyl mit 5 oder 6 Ringgliedern und 1 bis 3 Heteroatomen, wie Stickstoff, Sauerstoff und/oder Schwefel, steht, wobei das Heterocyclyl einfach oder zweifach substituiert sein kann durch Halogen, Alkyl mit 1 bis 4 Kohlenstoffatomen, Cyano, Nitro und/oder Cycloalkyl mit 3 bis 6 Kohlenstoffatomen,
R² für Wasserstoff oder Alkyl mit 1 bis 4 Kohlenstoffatomen steht, oder
R¹ und R² gemeinsam mit dem Stickstoffatom, an das sie gebunden sind, für einen gesättigten oder ungesättigten heterocyclischen Ring mit 3 bis 6 Ringgliedern stehen, wobei der Heterocyclus ein weiteres Stickstoff-, Sauerstoff- oder Schwefelatom als Ringglied enthalten kann und wobei der Heterocyclus bis zu dreifach substituiert sein kann durch Fluor, Chlor, Brom, Alkyl mit 1 bis 4 Kohlenstoffatomen und/oder Halogenalkyl mit 1 bis 4 Kohlenstoffatomen und 1 bis 9 Fluor- und/oder Chloratomen,
R³ für Wasserstoff, Fluor, Chlor, Brom, Iod, Alkyl mit 1 bis 4 Kohlenstoffatomen, Halogenalkyl mit 1 bis 4 Kohlenstoffatomen und 1 bis 9 Halogenatomen oder für Cycloalkyl mit 3 bis 6 Kohlenstoffatomen steht,
R⁴ für einen Rest der Formel 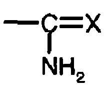 steht, worin
X für ein Sauerstoffatom, eine HN-Gruppe oder eine HO-N-Gruppe steht,
oder
R⁴ für einen Rest der Formel 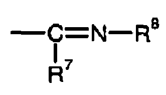 steht, worin
R⁷ für Wasserstoff oder Alkyl mit 1 bis 4 Kohlenstoffatomen steht und
R⁸ für Hydroxy, Alkyl mit 1 bis 4 Kohlenstoffatomen steht, wobei jeder der Alkyl-Reste, einfach oder zweifach substituiert sein kann durch Alkoxy mit 1 bis 4 Kohlenstoffatomen, Alkylcarbonyl mit 1 bis 3 Kohlenstoffatomen im Alkylteil und/oder Alkoxycarbonyl mit 1 bis 3 Kohlenstoffatomen im Alkoxyteil, oder
R⁸ für Phenyl steht, das einfach bis dreifach, gleichartig oder verschieden substituiert sein kann durch Alkyl mit 1 bis 4 Kohlenstoffatomen, Alkoxy mit 1 bis 4 Kohlenstoffatomen, Halogen, Nitro und/oder Halogenalkyl mit 1 bis 4 Kohlenstoffatomen und 1 bis 5 Halogenatomen, oder
R⁸ für Phenylamino steht, das einfach bis dreifach, gleichartig oder verschieden substituiert sein kann durch Alkyl mit 1 bis 4 Kohlenstoffatomen, Alkoxy mit 1 bis 4 Kohlenstoffatomen, Halogen, Nitro und/oder Halogenalkyl mit 1 bis 4 Kohlenstoffatomen und 1 bis 5 Halogenatomen,
R⁵ für Fluor, Chlor, Brom. Alkoxy mit 1 bis 4 Kohlenstoffatomen, Alkylthio mit 1 bis 4 Kohlenstoffatomen, Alkylsulfinyl mit 1 bis 4 Kohlenstoffatomen oder Alkylsulfonyl mit 1 bis 4 Kohlenstoffatomen steht, und
R⁶ für Phenyl steht, das einfach bis vierfach, gleichartig oder verschieden substituiert sein kann durch Halogen, Cyano, Nitro, Amino, Hydroxy, Formyl, Carboxy, Carbamoyl, Thiocarbamoyl;
jeweils geradkettiges oder verzweigtes Alkyl, Alkoxy, Alkylthio, Alkylsulfinyl oder Alkylsulfonyl mit jeweils 1 bis 6 Kohlenstoffatomen;
jeweils geradkettiges oder verzweigtes Alkenyl oder Alkenyloxy mit jeweils 2 bis 6 Kohlenstoffatomen;
jeweils geradkettiges oder verzweigtes Halogenalkyl, Halogenalkoxy. Halogenalkylthio, Halogenalkylsulfinyl oder Halogenalkylsulfonyl mit jeweils 1 bis 6 Kohlenstoffatomen und 1 bis 13 gleichen oder verschiedenen Halogenatomen;
jeweils geradkettiges oder verzweigtes Halogenalkenyl oder Halgoenalkenyloxy mit jeweils 2 bis 6 Kohlenstoffatomen und 1 bis 11 gleichen oder verschiedenen Halogenatomen;
jeweils geradkettiges oder verzweigtes Alkylamino, Dialkylamino, Alkylcarbonyl, Alkylcarbonyloxy, Alkoxycarbonyl, Alkylsulfonyloxy, Hydroximinoalkyl oder Alkoximinoalkyl mit jeweils 1 bis 6 Kohlenstoffatomen in den einzelnen Alkylteilen;
Cycloalkyl mit 3 bis 6 Kohlenstoffatomen,
in 2,3-Position verknüpftes 1,3-Propandiyl, 1,4-Butandiyl, Methylendioxy (-O-CH₂-O) oder 1,2-Ethylendioxy (-O-CH₂-CH₂-O-), wobei die Reste einfach oder mehrfach, gleichartig oder verschieden substituiert sein können durch Halgoen, Alkyl mit 1 bis 4 Kohlenstoffatomen und/oder Halogenalkyl mit 1 bis 4 Kohlenstoffatomen und 1 bis 9 gleichen oder verschiedenen Halogenatomen.

2. Pyrazolopyrimidine der Formel (I) gemäß Anspruch 1, in denen
R¹ für Wasserstoff oder einen Rest der Formel 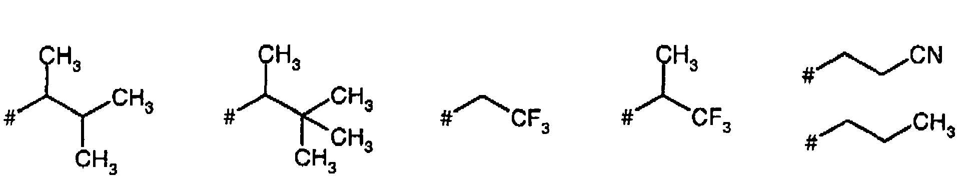 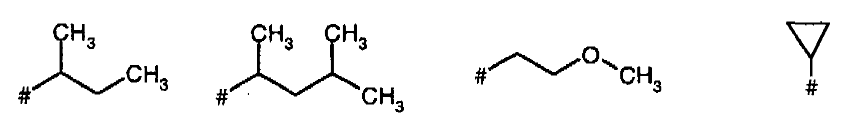 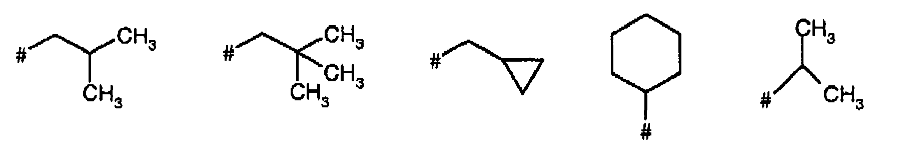 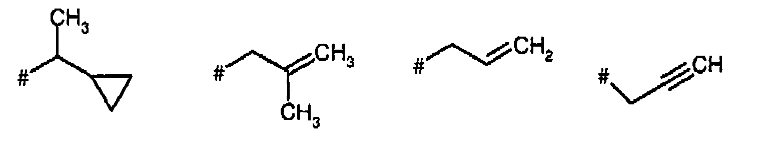 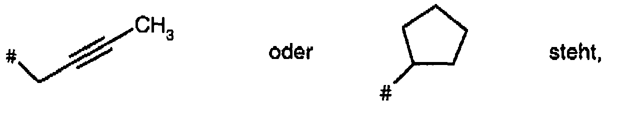 wobei # die Anknüpfungsstelle markiert,
R² für Wasserstoff, Methyl, Ethyl oder Propyl steht, oder
R¹ und R² gemeinsam mit dem Stickstoffatom, an das sie gebunden sind, für Pyrrolidinyl, Piperidinyl, Morpholinyl, Thiomorpholinyl, Piperazinyl, 3,6-Dihydro-1(2H)-piperidinyl oder Tetrahydro-1(2H)-pyridazinyl stehen, wobei diese Reste durch 1 bis 3 Fluoratome, 1 bis 3 Methylgruppen und/oder Trifluormethyl substituiert sein können,
oder
R¹ und R² gemeinsam mit dem Stickstoffatom, an das sie gebunden sind, für einen Rest der Formel 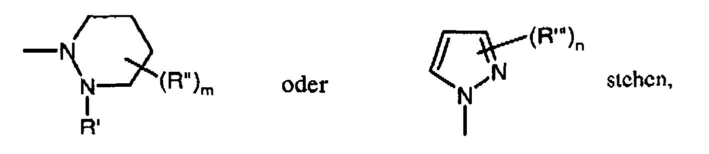 worin
R' für Wasserstoff oder Methyl steht,
R" für Methyl, Ethyl, Fluor, Chlor oder Trifluormethyl steht,
m für die Zahlen 0, 1, 2 oder 3 steht, wobei R" für gleiche oder verschiedene Reste steht, wenn m für 2 oder 3 steht,
R'" für Methyl, Ethyl, Fluor, Chlor oder Trifluormethyl steht
und
n für die Zahlen 0, 1, 2 oder 3 steht, wobei R"' für gleiche oder verschiedene Reste steht, wenn n für 2 oder 3 steht,
R³ für Wasserstoff, Fluor, Chlor, Brom, Iod, Methyl, Ethyl, Isopropyl, Cyclopropyl, Cyclobutyl, Cyclopentyl, Cyclohexyl, Trifluormethyl, 1-Trifluormethyl-2,2,2-trifluorethyl oder Heptafluorisopropyl steht,
R⁴ für einen Rest der Formel 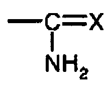 steht, worin
X für ein Sauerstoffatom, ein Schwefelatom, eine HN-Gruppe oder eine HO-N-Gruppe steht, oder
R⁴ für einen Rest der Formel 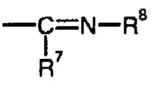 steht, worin
R⁷ für Wasserstoff, Methyl oder Ethyl steht und
R⁸ für Alkyl mit 1 oder 2 Kohlenstoffatomen steht, wobei jeder dieser Alkyl-Reste substituiert sein kann durch Methoxy, Ethoxy, Methylcarbonyl, Ethylcarbonyl, Methoxycarbonyl oder Ethoxycarbonyl, oder
R⁸ für Phenyl steht, das einfach bis dreifach, gleichartig oder verschieden substituiert sein kann durch Methyl, Ethyl, Methoxy, Ethoxy, Fluor, Chlor, Brom, Nitro und/oder Trifluormethyl, oder
R⁸ für Phenylamino steht, das einfach bis dreifach, gleichartig oder verschieden substituiert sein kann durch Methyl, Ethyl, Methoxy, Ethoxy, Fluor, Chlor, Brom, Nitro und/oder Trifluormethyl,
R⁵ für Fluor, Chlor, Brom, Methoxy, Ethoxy, Methylthio, Methylsulfinyl der Methylsulfonyl steht, und
R⁶ für Phenyl steht, das einfach bis dreifach, gleichartig oder verschieden substitiert sein kann durch Fluor, Chlor, Brom, Cyano, Nitro, Formyl, Methyl, Ethyl, n- oder i- Propyl, n-, i-, s- oder t-Butyl, Allyl, Propargyl, Methoxy, Ethoxy, n- oder i-Propoxy, Methylthio, Ethylthio, n- oder i-Propylthio, Methylsulfinyl, Ethylsulfinyl, Methylsulfonyl, Ethylsulfonyl, Allyloxy, Propargyloxy, Trifluormethyl, Trifluorethyl, Difluormethoxy, Trifluormethoxy, Difluorchlormethoxy, Trifluorethoxy, Difluormethylthio, Difluorchlorniethylthio, Trifluormethylthio, Trifluormethylsulfinyl, Trifluormethylsulfonyl, Trichlorethinyloxy, Trifluorethinyloxy, Chlorallyloxy, Iodpropargyloxy, Methylamino, Ethylamino, n- oder i-Propylamino. Dimethylamino. Diethylamino, Acetyl, Propionyl, Acetyloxy, Methoxycarbonyl, Ethoxycarbonyl, Hydroximinomethyl, Hydroximinoethyl, Methoximinomethyl, Ethoximinomethyl, Methoximinoethyl, Ethoximinoethyl, Cyclopropyl, Cyclobutyl, Cyclopentyl oder Cyclohexyl,
in 2,3-Position verknüpftes 1,3-Propandiyl, Methylendioxy (-O-CH₂-O-) oder 1,2-Ethylendioxy (-O-CH₂-CH₂-O-), wobei diese Reste einfach oder mehrfach, gleichartig oder verschieden substituiert sein können durch Fluor, Chlor, Methyl, Ethyl, n-Propyl, i-Propyl und/oder Trifluormethyl.

3. Pyrazolopyrimidine der Formel (I) gemäß Anspruch 1 oder 2, in denen
R⁵ für Fluor, Chlor, Brom, Methoxy oder Methylthio steht und
R⁶ für 2,4-, 2,5- oder 2,6-disubstituiertes Phenyl, oder 2-substituiertes Phenyl oder für 2,4,6-trisubstituiertes Phenyl steht, wobei die Substituenten gewählt sind aus der Gruppe
Fluor, Chlor, Brom, Cyano, Nitro, Formyl. Methyl, Ethyl, n- oder i-Propyl, n-, i-, s- oder t-Butyl, Allyl, Propargyl, Methoxy, Ethoxy, n- oder i-Propoxy, Methylthio, Ethylthio, n- oder i-Propylthio, Methylsulfinyl, Ethylsulfinyl, Methylsulfonyl, Ethylsulfonyl, Allyloxy, Propargyloxy, Trifluormethyl, Trifluorethyl, Difluormethoxy, Tritluormethoxy, Difluorchlormethoxy, Trifluorethoxy, Difluormethylthio, Difluorchlormethylthio, Trifluormethylthio, Trifluormethylsulfinyl, Trifluormethylsulfonyl, Trichlorethinyloxy, Trifluorethinyloxy, Chlorallyloxy, Iodpropargyloxy, Methylamino, Ethylamino, n- oder i-Propylamino, Dimethylamino, Diethylamino, Acetyl, Propionyl, Acetyloxy, Methoxycarbonyl, Ethoxycarbonyl, Hydroximinomethyl, Hydroximinoethyl, Methoximinomethyl, Ethoximinomethyl, Methoximinoethyl, Ethoximinoethyl, Cyclopropyl, Cyclobutyl, Cyclopentyl, Cyclohexyl,
in 2,3-Position verknüpftes 1,3-Propandiyl, Methylendioxy (-O-CH₂-O-) und 1,2-Ethylendioxy (-O-CH₂-CH₂-O-), wobei diese Reste einfach oder mehrfach, gleichartig oder verschieden substituiert sein können durch Fluor, Chlor, Methyl, Ethyl, n-Propyl, i-Propyl und/oder Trifluormethyl.

4. Verfahren zur Herstellung von Pyrazolopyrimidinen der Formel (I) gemäß einem oder mehreren der Ansprüche 1 bis 3, **dadurch gekennzeichnet, dass** man
a) Cyano-Verbindungen der Formel 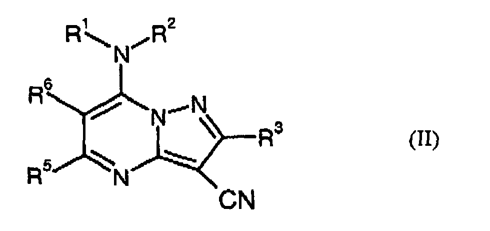 in welcher
R¹, R², R³, R⁵ und R⁶ die oben angegebenen Bedeutungen haben,
entweder
α) mit Säuren und Wasser gegebenenfalls in Gegenwart eines Verdünnungsmittels umsetzt,
oder
β) mit Hydroxylamin oder einem Hyroxylauunonium-Salz in Gegenwart eines Verdünnungsmittels und gegebenenfalls in Gegenwart eines Katalysators umsetzt,
oder
γ) mit Ammoniumchlorid in Gegenwart einer Base und in Gegenwart eines Verdünnungsmittels umsetzt,
oder
b) Carbonyl-Verbindungen der Formel 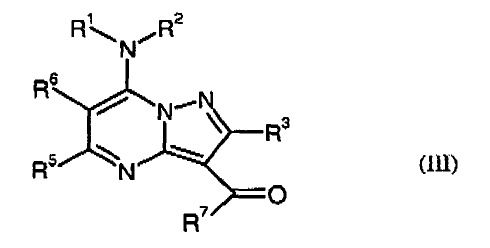
in welcher
R¹, R², R³, R⁵, R⁶ und R⁷ die oben angegebenen Bedeutungen haben,
mit Amino-Verbindungen der Formel
H₂N-R⁸ (IV)
in welcher
R⁸ die oben angegebenen Bedeutungen hat,
in Gegenwart eines Verdünnungsmittels und gegebenenfalls in Gegenwart eines Katalysators umsetzt, wobei die Amino-Verbindungen der Formel (IV) auch in Form ihrer Säureadditions-Salze eingesetzt werden können.

5. Mittel zur Bekämpfung von unerwünschten Mikroorganismen, **gekennzeichnet durch** einen Gehalt an mindestens einem Pyrazolopyrimidin der Formel (1) gemäß einem oder mehreren der Ansprüche 1 bis 3 neben Streckmitteln und/oder oberflächenaktiven Stoffen.

6. Mittel gemäß Anspruch 5, enthaltend mindestens einen weiteren fungiziden oder insektiziden Wirkstoff.

7. Verwendung von Pyrazolopyrimidinen der Formel (I) gemäß einem oder mehreren der Ansprüche 1 bis 3 zur Bekämpfung von unerwünschten Mikroorganismen im Pflanzen- und im Materialschutz.

8. Verfahren zur Bekämpfung von unerwünschten Mikroorganismen, **dadurch gekennzeichnet, dass** man Pyrazolopyrimidine der Formel (I) gemäß einem oder mehreren der Ansprüche 1 bis 3 auf die unerwünschten Mikroorganismen und/oder deren Lebensraum ausbringt.

9. Verfahren zur Herstellung von Mitteln zur Bekämpfung von unerwünschten Mikroorganismen, **dadurch gekennzeichnet, dass** man Pyrazolopyrimidine der Formel (I) gemäß einem oder mehreren der Ansprüche 1 bis mit Streckmitteln und/oder oberflächenaktiven Stoffen vermischt.

## Claims

1. Pyrazolopyrimidines of the formula 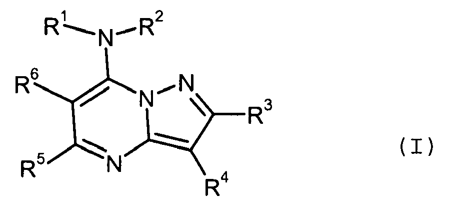 in which
R¹ represents hydrogen, alkyl having 1 to 6 carbon atoms which may be mono- to penta-substituted by identical or different substituents from the group consisting of halogen, cyano, hydroxyl, alkoxy having 1 to 4 carbon atoms and cycloalkyl having 3 to 6 carbon atoms,
R¹ represents alkenyl having 2 to 6 carbon atoms which may be mono- to trisubstituted by identical or different substituents from the group consisting of halogen, cyano, hydroxyl, alkoxy having 1 to 4 carbon atoms and cycloalkyl having 3 to 6 carbon atoms, or
R¹ represents alkynyl having 3 to 6 carbon atoms which may be mono- to trisubstituted by identical or different substituents from the group consisting of halogen, cyano, alkoxy having 1 to 4 carbon atoms and cycloalkyl having 3 to 6 carbon atoms, or
R¹ represents cycloalkyl having 3 to 6 carbon atoms which may be mono- to trisubstituted by identical or different substituents from the group consisting of halogen and alkyl having 1 to 4 carbon atoms, or
R¹ represents saturated or unsaturated heterocyclyl having 5 or 6 ring members and 1 to 3 hetero atoms, such as nitrogen, oxygen and/or sulfur, where the heterocyclyl may be mono- or disubstituted by halogen, alkyl having 1 to 4 carbon atoms, cyano, nitro and/or cycloalkyl having 3 to 6 carbon atoms,
R² represents hydrogen or alkyl having 1 to 4 carbon atoms, or
R¹and R² together with the nitrogen atom to which they are attached represent a saturated or unsaturated heterocyclic ring having 3 to 6 ring members, where the heterocycle may contain a further nitrogen, oxygen or sulfur atom as ring member and where the heterocycle may be substituted up to three times by fluorine, chlorine, bromine, alkyl having 1 to 4 carbon atoms and/or haloalkyl having 1 to 4 carbon atoms and 1 to 9 fluorine and/or chlorine atoms,
R³ represents hydrogen, fluorine, chlorine, bromine, iodine, alkyl having 1 to 4 carbon atoms, haloalkyl having 1 to 4 carbon atoms and 1 to 9 halogen atoms or represents cycloalkyl having 3 to 6 carbon atoms,
R⁴ represents a radical of the formula 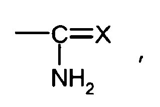 in which
X represents an oxygen atom, an HN group or an HO-N group,
or
R⁴ represents a radical of the formula 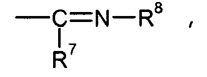 in which
R⁷ represents hydrogen or alkyl having 1 to 4 carbon atoms and
R⁸ represents hydroxyl, alkyl having 1 to 4 carbon atoms, where each of the alkyl radicals may be mono- or disubstituted by alkoxy having 1 to 4 carbon atoms, alkylcarbonyl having 1 to 3 carbon atoms in the alkyl moiety and/or alkoxycarbonyl having 1 to 3 carbon atoms in the alkoxy moiety, or
R⁸ represents phenyl which may be mono- to trisubstituted by identical or different substituents from the group consisting of alkyl having 1 to 4 carbon atoms, alkoxy having 1 to 4 carbon atoms, halogen, nitro and haloalkyl having 1 to 4 carbon atoms and 1 to 5 halogen atoms, or
R⁸ represents phenylamino which may be mono- to trisubstituted by identical or different substituents from the group consisting of alkyl having 1 to 4 carbon atoms, alkoxy having 1 to 4 carbon atoms, halogen, nitro and haloalkyl having 1 to 4 carbon atoms and 1 to 5 halogen atoms,
R⁵ represents fluorine, chlorine, bromine, alkoxy having 1 to 4 carbon atoms, alkylthio having 1 to 4 carbon atoms, alkylsulfinyl having 1 to 4 carbon atoms or alkylsulfonyl having 1 to 4 carbon atoms, and
R⁶ represents phenyl which may be mono- to tetrasubstituted by identical or different substituents from the group consisting of halogen, cyano, nitro, amino, hydroxyl, formyl, carboxyl, carbamoyl, thiocarbamoyl;
in each case straight-chain or branched alkyl, alkoxy, alkylthio, alkylsulfinyl or alkylsulfonyl having in each case 1 to 6 carbon atoms;
in each case straight-chain or branched alkenyl or alkenyloxy having in each case 2 to 6 carbon atoms;
in each case straight-chain or branched haloalkyl, haloalkoxy, haloalkylthio, haloalkylsulfinyl or haloalkylsulfonyl having in each case 1 to 6 carbon atoms and 1 to 13 identical or different halogen atoms;
in each case straight-chain or branched haloalkenyl or haloalkenyloxy having in each case 2 to 6 carbon atoms and 1 to 11 identical or different halogen atoms;
in each case straight-chain or branched alkylamino, dialkylamino, alkylcarbonyl, alkylcarbonyloxy, alkoxycarbonyl, alkylsulfonyloxy, hydroximinoalkyl or alkoximinoalkyl having in each case 1 to 6 carbon atoms in the individual alkyl moieties;
cycloalkyl having 3 to 6 carbon atoms,
2,3-attached 1,3-propanediyl, 1,4-butanediyl, methylenedioxy (-O-CH₂-O) or 1,2-ethylenedioxy (-O-CH₂-CH₂-O-), where the radicals may be mono- or polysubstituted by identical or different substituents from the group consisting of halogen, alkyl having 1 to 4 carbon atoms and haloalkyl having 1 to 4 carbon atoms and 1 to 9 identical or different halogen atoms.

2. Pyrazolopyrimidines of the formula (I) according to Claim 1, in which
R¹ represents hydrogen or a radical of the formula 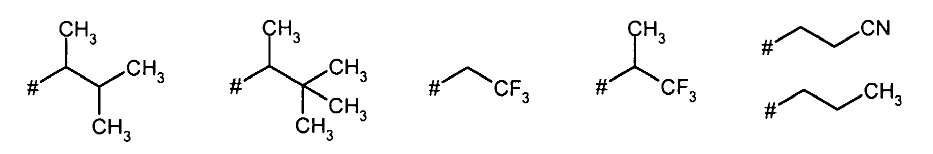 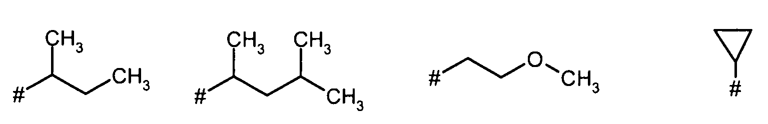 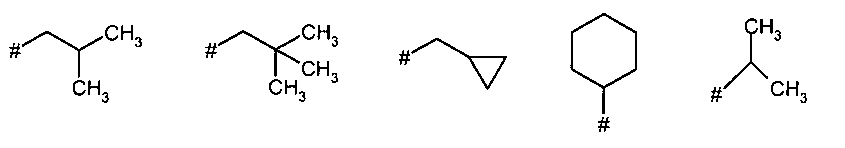 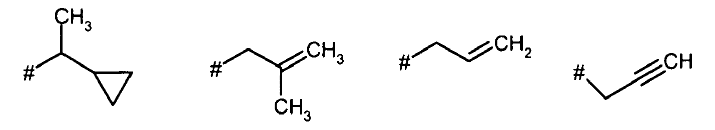 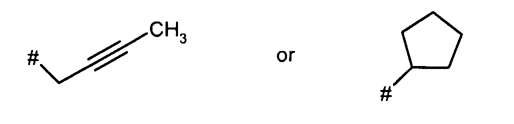 where # denotes the point of attachment,
R² represents hydrogen, methyl, ethyl or propyl,
or
R¹ and R² together with the nitrogen atom to which they are attached represent pyrrolidinyl, piperidinyl, morpholinyl, thiomorpholinyl, piperazinyl, 3,6-dihydro-1(2H)-piperidinyl or tetrahydro-1(2H)-pyridazinyl, where these radicals may be substituted by 1 to 3 fluorine atoms, 1 to 3 methyl groups and/or trifluoromethyl,
or
R¹ and R² together with the nitrogen atom to which they are attached represent a radical of the formula 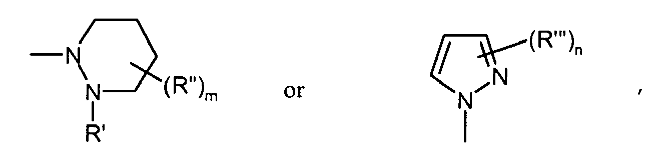 in which
R' represents hydrogen or methyl,
R" represents methyl, ethyl, fluorine, chlorine or trifluoromethyl,
m represents the number 0, 1, 2 or 3, where R" represents identical or different radicals if m represents 2 or 3,
R'" represents methyl, ethyl, fluorine, chlorine or trifluoromethyl
and
n represents the number 0, 1, 2 or 3, where R'" represents identical or different radicals if n represents 2 or 3,
R³ represents hydrogen, fluorine, chlorine, bromine, iodine, methyl, ethyl, isopropyl, cyclopropyl, cyclobutyl, cyclopentyl, cyclohexyl, trifluoromethyl, 1-trifluoromethyl-2,2,2-trifluoroethyl or heptafluoroisopropyl,
R⁴ represents a radical of the formula 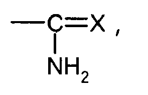 in which
X represents an oxygen atom, a sulfur atom, an HN group or an HO-N group, or
R⁴ represents a radical of the formula 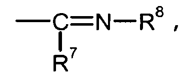 in which
R⁷ represents hydrogen, methyl or ethyl and
R⁸ represents alkyl having 1 or 2 carbon atoms, where each of these alkyl radicals may be substituted by methoxy, ethoxy, methylcarbonyl, ethylcarbonyl, methoxycarbonyl or ethoxycarbonyl, or
R⁸ represents phenyl which may be mono- to trisubstituted by identical or different substituents from the group consisting of methyl, ethyl, methoxy, ethoxy, fluorine, chlorine, bromine, nitro and trifluoromethyl, or
R⁸ represents phenylamino which may be mono- to trisubstituted by identical or different substituents from the group consisting of methyl, ethyl, methoxy, ethoxy, fluorine, chlorine, bromine, nitro and trifluoromethyl,
R⁵ represents fluorine, chlorine, bromine, methoxy, ethoxy, methylthio, methylsulfinyl or methylsulfonyl, and
R⁶ represents phenyl which may be mono- to trisubstituted by identical or different substituents from the group consisting of fluorine, chlorine, bromine, cyano, nitro, formyl, methyl, ethyl, n- or i-propyl, n-, i-, s- or t-butyl, allyl, propargyl, methoxy, ethoxy, n- or i-propoxy, methylthio, ethylthio, n- or i-propylthio, methylsulfinyl, ethylsulfinyl, methylsulfonyl, ethylsulfonyl, allyloxy, propargyloxy, trifluoromethyl, trifluoroethyl, difluoromethoxy, trifluoromethoxy, difluorochloromethoxy, trifluoroethoxy, difluoromethylthio, difluorochloromethylthio, trifluoromethylthio, trifluoromethylsulfinyl, trifluoromethylsulfonyl, trichloroethynyloxy, trifluoroethynyloxy, chloroallyloxy, iodopropargyloxy, methylamino, ethylamino, n-or i-propylamino, dimethylamino, diethylamino, acetyl, propionyl, acetyloxy, methoxycarbonyl, ethoxycarbonyl, hydroximinomethyl, hydroximinoethyl, methoximinomethyl, ethoximinomethyl, methoximinoethyl, ethoximinoethyl, cyclopropyl, cyclobutyl, cyclopentyl or cyclohexyl, 2,3-attached 1,3-propanediyl, methylenedioxy (-O-CH₂-O-) or 1,2-ethylenedioxy (-O-CH₂-CH₂-O-), where these radicals may be mono- or polysubstituted by identical or different substituents from the group consisting of fluorine, chlorine, methyl, ethyl, n-propyl, i-propyl and trifluoromethyl.

3. Pyrazolopyrimidines of the formula (I) according to Claim 1 or 2, in which
R⁵ represents fluorine, chlorine, bromine, methoxy or methylthio and
R⁶ represents 2,4-, 2,5- or 2,6-disubstituted phenyl or 2-substituted phenyl or represents 2,4,6-trisubstituted phenyl, the substituents being selected from the group consisting of fluorine, chlorine, bromine, cyano, nitro, formyl, methyl, ethyl, n- or i-propyl, n-, i-, s- or t-butyl, allyl, propargyl, methoxy, ethoxy, n- or i-propoxy, methylthio, ethylthio, n- or i-propylthio, methylsulfinyl, ethylsulfinyl, methylsulfonyl, ethylsulfonyl, allyloxy, propargyloxy, trifluoromethyl, trifluoroethyl, difluoromethoxy, trifluoromethoxy, difluorochloromethoxy, trifluoroethoxy, difluoromethylthio, difluorochloromethylthio, trifluoromethylthio, trifluoromethylsulfinyl, trifluoromethylsulfonyl, trichloroethynyloxy, trifluoroethynyloxy, chloroallyloxy, iodopropargyloxy, methylamino, ethylamino, n- or i-propylamino, dimethylamino, diethylamino, acetyl, propionyl, acetyloxy, methoxycarbonyl, ethoxycarbonyl, hydroximinomethyl, hydroximinoethyl, methoximinomethyl, ethoximinomethyl, methoximinoethyl, ethoximinoethyl, cyclopropyl, cyclobutyl, cyclopentyl, cyclohexyl,
2,3-attached 1,3-propanediyl, methylenedioxy (-O-CH₂-O-) and 1,2-ethylenedioxy (-O-CH₂-CH₂-O-), where these radicals may be mono- or polysubstituted by identical or different substituents from the group consisting of fluorine, chlorine, methyl, ethyl, n-propyl, i-propyl and trifluoromethyl.

4. Process for preparing pyrazolopyrimidines of the formula (I) according to one or more of Claims 1 to 3, **characterized in that**
a) cyano compounds of the formula 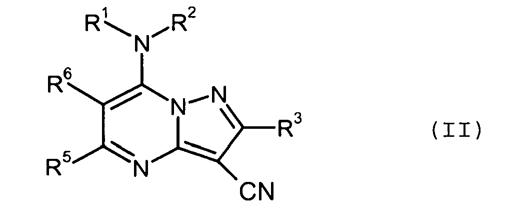 in which
R¹, R², R³, R⁵ and R⁶ are as defined above are either
α) reacted with acids and water, if appropriate in the presence of a diluent,
or
β) reacted with hydroxylamine or a hydroxylammonium salt in the presence of a diluent and, if appropriate, in the presence of a catalyst,
or
γ) reacted with ammonium chloride in the presence of a base and in the presence of a diluent,
or
b) carbonyl compounds of the formula 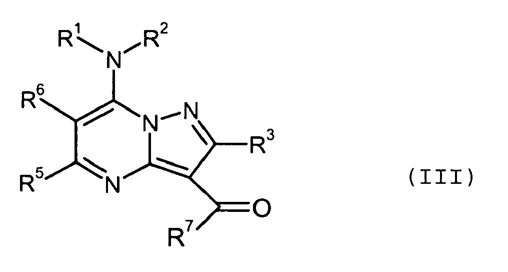 in which
R¹, R², R³, R⁵, R⁶ and R⁷ are as defined above are reacted with amino compounds of the formula
H₂N-R⁸ (IV)
in which
R⁸ is as defined above,
in the presence of a diluent and, if appropriate, in the presence of a catalyst, where the amino compounds of the formula (IV) may also be employed in the form of their acid addition salts.

5. Compositions for controlling unwanted microorganisms, **characterized in that** they comprise at least one pyrazolopyrimidine of the formula (I) according to one or more of Claims 1 to 3, in addition to extenders and/or surfactants.

6. Compositions according to Claim 5, comprising at least one further fungicidally or insecticidally active compound.

7. Use of pyrazolopyrimidines of the formula (I) according to one or more of Claims 1 to 3 for controlling unwanted microorganisms in crop protection and in the protection of materials.

8. Method for controlling unwanted microorganisms, **characterized in that** pyrazolopyrimidines of the formula (I) according to one or more of Claims 1 to 3 are applied to the unwanted microorganisms and/or their habitat.

9. Process for preparing compositions for controlling unwanted microorganisms, **characterized in that** pyrazolopyrimidines of the formula (I) according to one or more of Claims 1 to 3 are mixed with extenders and/or surfactants.

## Revendications

1. Pyrazolopyrimidines de formule 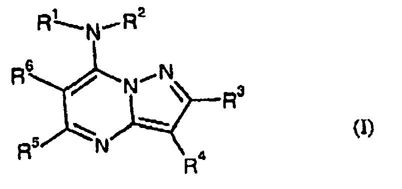 dans lesquelles
R¹ représente l'hydrogène, un reste alkyle ayant 1 à 6 atomes de carbone, qui peut être substitué une à cinq fois identiques ou différentes par un radical halogéno, cyano, hydroxy, alkoxy ayant 1 à 4 atomes de carbone et/ou cycloalkyle ayant 3 à 6 atomes de carbone,
R¹ représente un reste alcényle ayant 2 à 6 atomes de carbone, qui peut être substitué une à trois fois identiques ou différentes par un radical halogéno, cyano, hydroxy, alkoxy ayant 1 à 4 atomes de carbone et/ou cycloalkyle ayant 3 à 6 atomes de carbone, ou bien
R¹ représente un reste alcynyle ayant 3 à 6 atomes de carbone, qui peut être substitué une à trois fois identiques ou différentes par un radical halogéno, cyano, alkoxy ayant 1 à 4 atomes de carbone et/ou cycloalkyle ayant 3 à 6 atomes de carbone, ou bien
R¹ représente un reste cycloalkyle ayant 3 à 6 atomes de carbone, qui peut être substitué une à trois fois identiques ou différentes par un radical halogéno et/ou alkyle ayant 1 à 4 atomes de carbone, ou bien
R¹ représente un reste hétérocyclyle saturé ou non saturé pentagonal ou hexagonal ayant 1 à 3 hétéroatomes tels que l'azote, l'oxygène et/ou le soufre, le reste hétérocyclyle pouvant être substitué une ou deux fois par un radical halogéno, alkyle ayant 1 à 4 atomes de carbone, cyano, nitro et/ou cycloalkyle ayant 3 à 6 atomes de carbone,
R² représente l'hydrogène ou un reste alkyle ayant 1 à 4 atomes de carbone, ou bien
R¹ et R² forment, conjointement avec l'atome d'azote auquel ils sont liés, un noyau hétérocyclique saturé ou non saturé trigonal à hexagonal, l'hétérocycle pouvant contenir comme phénon un autre atome d'azote, d'oxygène ou de soufre, et l'hétérocycle pouvant être substitué jusqu'à trois fois par un radical fluoro, chloro, bromo, alkyle ayant 1 à 4 atomes de carbone et/ou halogénalkyle ayant 1 à 4 atomes de carbone et 1 à 9 atomes de fluor et/ou de chlore,
R³ représente l'hydrogène, le fluor, le chlore, le brome, l'iode, un reste alkyle ayant 1 à 4 atomes de carbone, halogénalkyle ayant 1 à 4 atomes de carbone et 1 à 9 atomes d'halogène ou un reste cycloalkyle ayant 3 à 6 atomes de carbone,
R⁴ représente un reste de formule 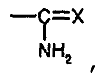 dans laquelle
X représente un atome d'oxygène, un groupe HN ou un groupe HO-N,
ou bien
R⁴ représente un reste de formule 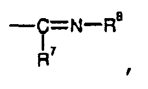 dans laquelle
R⁷ représente l'hydrogène ou un reste alkyle ayant 1 à 4 atomes de carbone, et
R⁸ représente un groupe hydroxy, un reste alkyle ayant 1 à 4 atomes de carbone, chacun des restes alkyle pouvant être substitué une ou deux fois par un radical alkoxy ayant 1 à 4 atomes de carbone, alkylcarbonyle ayant 1 à 3 atomes de carbone dans la partie alkyle et/ou alkoxycarbonyle ayant 1 à 3 atomes de carbone dans la partie alkoxy, ou bien
R⁸ représente un reste phényle qui peut être substitué une à trois fois identiques ou différentes par un radical alkyle ayant 1 à 4 atomes de carbone, alkoxy ayant 1 à 4 atomes de carbone, halogéno, nitro et/ou halogénalkyle ayant 1 à 4 atomes de carbone et 1 à 5 atomes d'halogène, ou bien
R⁸ représente un reste phénylamino qui peut être substitué une à trois fois identiques ou différentes par un radical alkyle ayant 1 à 4 atomes de carbone, alkoxy ayant 1 à 4 atomes de carbone, halogéno, nitro et/ou halogénalkyle ayant 1 à 4 atomes de carbone et 1 à 5 atomes d'halogène, et
R⁵ représente le fluor, le chlore, le brome, un reste alkoxy ayant 1 à 4 atomes de carbone, alkylthio ayant 1 à 4 atomes de carbone, alkylsulfinyle ayant 1 à 4 atomes de carbone ou alkylsulfonyle ayant 1 à 4 atomes de carbone, et
R⁶ représente un reste phényle qui peut être substitué une à quatre fois identiques ou différentes par un radical halogéno, cyano, nitro, amino, hydroxy, formyle, carboxy, carbamoyle, thiocarbamoyle ;
un reste alkyle, alkoxy, alkylthio, alkylsulfinyle ou alkylsulfonyle ayant chacun 1 à 6 atomes de carbone et chacun étant linéaire ou ramifié ;
un reste alcényle ou alcényloxy ayant chacun 2 à 6 atomes de carbone et chacun étant linéaire ou ramifié ;
un reste halogénalkyle, halogénalkoxy, halogénalkylthio, halogénalkylsulfinyle ou halogénalkylsulfonyle ayant chacun 1 à 6 atomes de carbone et 1 à 13 atomes d'halogène identiques ou différents et chacun étant linéaire ou ramifié ;
un reste halogénalcényle ou halogénalcényloxy ayant chacun 2 à 6 atomes de carbone et 1 à 11 atomes d'halogène identiques ou différents et chacun étant linéaire ou ramifié ;
un reste alkylamino, dialkylamino, alkylcarbonyle, alkylcarbonyloxy, alkoxycarbonyle, alkylsulfonyloxy, hydroximinoalkyle ou alkoximinoalkyle ayant chacun 1 à 6 atomes de carbone dans les parties alkyle individuelles et chacun étant linéaire ou ramifié ;
un reste cycloalkyle ayant 3 à 6 atomes de carbone, un reste, lié en position 2,3, 1,3-propanediyle, 1,4-butanediyle, méthylènedioxy (-O-CH₂-O) ou 1,2-éthylènedioxy (-O-CH₂-CH₂-O-), les restes pouvant être substitués une ou plusieurs fois identiques ou différentes par un radical halogéno, alkyle ayant 1 à 4 atomes de carbone et/ou halogénalkyle ayant 1 à 4 atomes de carbone et 1 à 9 atomes d'halogène identiques ou différents.

2. Pyrazolopyrimidines de formule (I) suivant la revendication 1, dans lesquelles
R¹ représente l'hydrogène ou un reste de formule 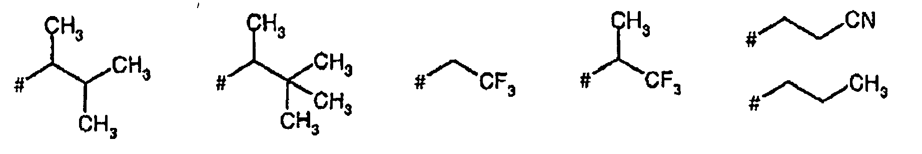 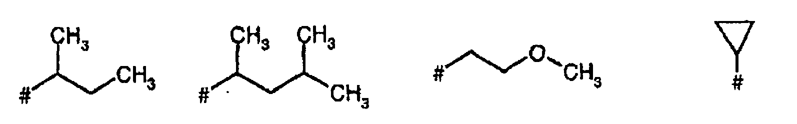 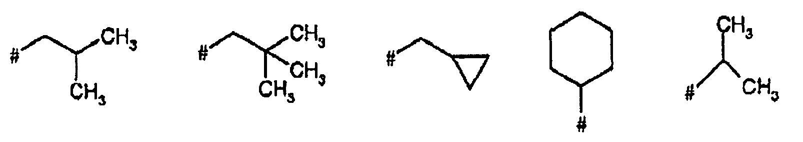 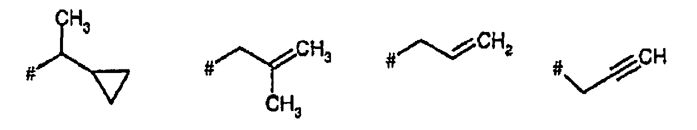 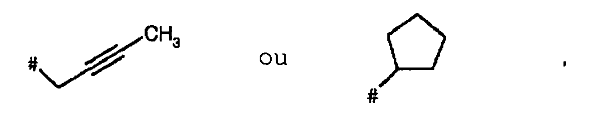
le signe # indiquant la position de rattachement,
R² représente l'hydrogène, un reste méthyle, éthyle ou propyle, ou bien
R¹ et R², conjointement avec l'atome d'azote auquel ils sont liés, forment un reste pyrrolidinyle, pipéridinyle, morpholinyle, thiomorpholinyle, pipérazinyle, 3,6-dihydro-1(2H)-pipéridinyle ou tétrahydro-1(2H)-pyridazinyle, ces restes pouvant être substitués par 1 à 3 atomes de fluor, 1 à 3 groupes méthyle et/ou un groupe trifluorométhyle,
ou bien
R¹ et R² forment, conjointement avec l'atome d'azote auquel ils sont liés, un reste de formule 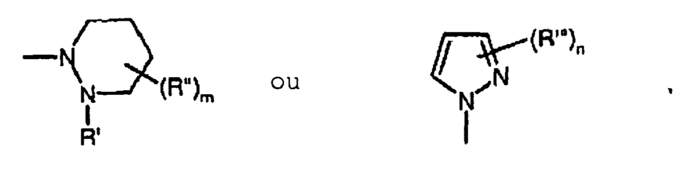
dans laquelle
R' représente l'hydrogène ou un reste méthyle,
R" représente un reste méthyle, éthyle, fluoro, chloro ou trifluorométhyle,
m représente les nombres 0, 1, 2 ou 3, R" représentant des restes identiques ou différents lorsque m a la valeur 2 ou 3,
R"' représente un reste méthyle, éthyle, fluoro, chloro ou trifluorométhyle,
et
n représente les nombres 0, 1, 2 ou 3, R"' représentant des restes identiques ou différents lorsque n a la valeur 2 ou 3,
R³ représente l'hydrogène, le fluor, le chlore, le brome, l'iode, un reste méthyle, éthyle, isopropyle, cyclopropyle, cyclobutyle, cyclopentyle, cyclohexyle, trifluorométhyle, 1-trifluorométhyl-2, 2, 2-trifluoréthyle ou heptafluorisopropyle,
R⁴ représente un reste de formule 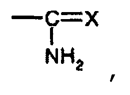 dans laquelle
X est un atome d'oxygène, un atome de soufre, un groupe HN ou un groupe HO-N, ou bien
R⁴ représente un reste de formule 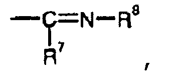 dans laquelle
R⁷ représente l'hydrogène, un reste méthyle ou éthyle, et
R⁸ représente un reste alkyle ayant 1 ou 2 atomes de carbone, chacun de ces restes alkyle pouvant être substitué par un radical méthoxy, éthoxy, méthylcarbonyle, éthylcarbonyle, méthoxycarbonyle ou éthoxycarbonyle, ou bien
R⁸ représente un reste phényle qui peut être substitué une à trois fois identiques ou différentes par un radical méthyle, éthyle, méthoxy, éthoxy, fluoro, chloro, bromo, nitro et/ou trifluorométhyle, ou bien
R⁸ représente un reste phénylamino qui peut être substitué une à trois fois identiques ou différentes par un radical méthyle, éthyle, méthoxy, éthoxy, fluoro, chloro, bromo, nitro et/ou trifluorométhyle,
R⁵ représente le fluor, le chlore, le brome, un reste méthoxy, éthoxy, méthylthio, méthylsulfinyle ou méthylsulfonyle, et
R⁶ représente un reste phényle qui peut être substitué une à trois fois identiques ou différentes par un radical fluoro, chloro, bromo, cyano, nitro, formyle, méthyle, éthyle, n-propyle, isopropyle, n-butyle, isobutyle, sec.-butyle, tertiobutyle, allyle, propargyle, méthoxy, éthoxy, n-propoxy, isopropoxy, méthylthio, éthylthio, n-propylthio, isopropylthio, méthylsulfinyle, éthylsulfinyle, méthylsulfonyle, éthylsulfonyle, allyloxy, propargyloxy, trifluorométhyle, trifluoréthyle, difluorométhoxy, trifluorométhoxy, difluorochlorométhoxy, trifluoréthoxy, difluorométhylthio, difluorochlorométhylthio, trifluorométhylthio, trifluorométhylsulfinyle, trifluorométhylsulfonyle, trichloréthynyloxy, trifluoréthylnyloxy, chlorallyloxy, iodopropargyloxy, méthylamino, éthylamino, n-propylamino, isopropylamino, diméthylamino, diéthylamino, acétyle, propionyle, acétyloxy, méthoxycarbonyle, éthoxycarbonyle, hydroximinométhyle, hydroximinoéthyle, méthoximinométhyle, éthoximinométhyle, méthoximinoéthyle, éthoximinoéthyle, cyclopropyle, cyclobutyle, cyclopentyle ou cyclohexyle, un reste, lié en position 2,3, 1,3-propanediyle, méthylènedioxy (-O-CH₂-O-) ou 1,2-éthylènedioxy (-O-CH₂-CH₂-O-), ces restes pouvant être substitués une ou plusieurs fois identiques ou différentes par un radical fluoro, chloro, méthyle, éthyle, n-propyle, isopropyle et/ou trifluorométhyle.

3. Pyrazolopyrimidines de formule (I) suivant la revendication 1 ou 2, dans lesquelles
R⁵ représente le fluor, le chlore, le brome, un reste méthoxy ou méthylthio, et
R⁶ représente un reste phényle disubstitué en position 2,4, 2,5 ou 2,6, ou un reste phényle substitué en position 2, ou un reste phényle trisubstitué en positions 2, 4, 6, les substituants étant choisis dans le groupe des substituants
fluor, chlore, brome, cyano, nitro, formyle, méthyle, éthyle, n-propyle, isopropyle, n-butyle, isobutyle, sec.-butyle, tertiobutyle, allyle, propargyle, méthoxy, éthoxy, n-propoxy, isopropoxy, méthylthio, éthylthio, n-propylthio, isopropylthio, méthylsulfinyle, éthylsulfinyle, méthylsulfonyle, éthylsulfonyle, allyloxy, propargyloxy, trifluorométhyle, trifluoréthyle, difluorométhoxy, trifluorométhoxy, difluorochlorométhoxy, trifluoréthoxy, difluorométhylthio, difluorochlorométhylthio, trifluorométhylthio, trifluorométhylsulfinyle, trifluorométhylsulfonyle, trichloréthynyloxy, trifluoréthynyloxy, chlorallyloxy, iodopropargyloxy, méthylamino, éthylamino, n-propylamino, isopropylamino, diméthylamino, diéthylamino, acétyle, propionyle, acétyloxy, méthoxycarbonyle, éthoxycarbonyle, hydroximinométhyle, hydroximinoéthyle, méthoximinométhyle, éthoximinoéméthyle, méthoximinoéthyle, éthoximinoéthyle, cyclopropyle, cyclobutyle, cyclopentyle, cyclohexyle, un reste, lié en positions 2,3, 1,3-propanediyle, méthylènedioxy (-O-CH₂-O-) et 1,2-éthylènedioxy (-O- CH₂-CH₂-O-), ces restes pouvant être substitués une ou plusieurs fois identiques ou différentes par un radical fluoro, chloro, méthyle, éthyle, n-propyle, isopropyle et/ou trifluorométhyle.

4. Procédé de production de pyrazolopyrimidines de formule (I) suivant une ou plusieurs des revendications 1 à 3, **caractérisé en ce que** :
a) des composés portant un groupe cyano, de formule 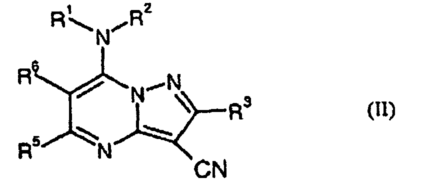 dans laquelle
R¹, R², R³, R⁵ et R⁶ ont les définitions indiquées ci-dessus, sont amenés à réagir
α) avec des acides et de l'eau, éventuellement en présence d'un diluant,
ou bien
β) avec l'hydroxylamine ou un sel d'hydroxylammonium en présence d'un diluant et, le cas échéant, en présence d'un catalyseur,
ou bien
γ) avec le chlorure d'ammonium en présence d'une base et en présence d'un diluant,
ou bien
b) des composés portant un groupe carbonyle, de formule 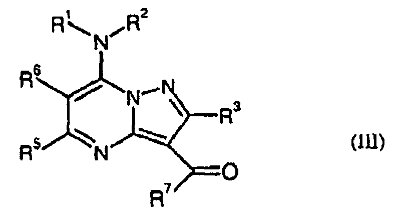
dans laquelle
R¹, R², R³, R⁵ et R⁶ et R⁷ ont les définitions indiquées ci-dessus,
sont amenés à réagir avec des composés aminé de formule
H₂N-R⁸ (IV)
dans laquelle
R⁸ a les définitions indiquées ci-dessus,
en présence d'un diluant et, le cas échéant, en présence d'un catalyseur, les composés aminés de formule (IV) pouvant aussi être utilisés sous forme de leurs sels d'additions d'acides.

5. Composition destinée à combattre des microorganismes indésirables, **caractérisée par** une teneur en au moins une pyrazolopyrimidine de formule (I) suivant une ou plusieurs des revendications 1 à 3, à côté de diluants et/ou d'agents tensioactifs.

6. Composition suivant la revendication 5, contenant au moins une autre substance active fongicide ou insecticide.

7. Utilisation de pyrazolopyrimidines de formule (I) suivant une ou plusieurs des revendications 1 à 3, pour combattre des microorganismes indésirables dans la protection des plantes et des matériaux.

8. Procédé de lutte contre des microorganismes indésirables, **caractérisé en ce qu'**on épand des pyrazolopyrimidines de formule (I) suivant une ou plusieurs des revendications 1 à 3 sur les microorganismes indésirables et/ou sur leur milieu.

9. Procédé de préparation de compositions destinées à combattre des microorganismes indésirables, **caractérisé en ce qu'**on mélange des pyrazolopyrimidines de formule (I) suivant une ou plusieurs des revendications 1 à 3, avec des diluants et/ou des agents tensioactifs.
